# EUROPEAN PATENT APPLICATION

(11) **EP 1 114 998 A2**
(43) Date of publication of application: **11.07.2001**
(21) Application number: 00203765.3
(22) Date of filing: 27.10.2000
(51) Int. Cl.: G01N 33/53

(54) **Method for determining the success rate of treatment of multiple sclerosis**

(30) Priority: 28.10.1999 EP 99203551; 30.03.2000 EP 00201167
(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Nagelkerken, Alexander Maria, 2334 CH Leiden (NL); Van Boxel-Dezaire, Anette Hendrika Hildegard, 2713 RX Zoetermeer (NL); Polman, Chris Hubert, 2051 HE Overveen (NL)
(74) Representative: Jorritsma, Ruurd

(57) **Abstract**

The invention relates to a method for following the progres and/or treatment of multiple sclerosis on the basis of at least one biological sample obtained from a person suffering from, or suspected to suffer from, multiple sclerosis, said method comprising the steps of:
a) providing a biological sample derived from a patient suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1, IL-12Rβ2 and/or IL-12p35 of said first biological sample; and optionally
c) comparing for one or more of the cytokines determined in step b) the value(s) obtained in step b) with a reference value.

The invention allows determination of the success rate of treatment of multiple sclerosis by discriminating between patients with multiple sclerosis. In particular, a distinction can be made between relapsing remitting multiple sclerosis, secondary progressive multiple sclerosis and primary progressive multiple sclerosis and healthy controls. More particularly the invention allows determination of the success rate of treatment of multiple sclerosis with interferon-beta (IFN-β) species such as IFN-β1b and IFN-β1a.

## Description

The subject invention relates to a method for following the progress and/or treatment of multiple sclerosis.

More specific it concerns a method for predicting and/or determining the chance of success of the treatment of patients suffering from multiple sclerosis. In particular the invention concerns a method for discriminating between different clinical subtypes of multiple sclerosis (MS).

In its broadest sense the invention relates to a method for following the progres and/or treatment of multiple sclerosis on the basis of at least one biological sample obtained from a person suffering from, or suspected to suffer from, multiple sclerosis, said method comprising the steps of:
a) providing a biological sample derived from a patient suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1, IL-12Rβ2 and/or IL-12p35 of said first biological sample; and optionally
c) comparing for one or more of the cytokines determined in step b) the value(s) obtained in step b) with a reference value.

It is known that in multiple sclerosis, different clinical phases/stages of the disease may occur. In particular, a distinction can be made between relapsing remitting multiple sclerosis (RR-MS), secondary progressive multiple sclerosis (SP-MS) and primary progressive multiple sclerosis (PP-MS).

In RR-MS, the patient by definition experiences the symptoms of MS - e.g. paralysis phenomena - only for a limited period of time, e.g. of about several weeks and sometimes even to about several months. The symptoms may then disappear (or at least be reduced) for some weeks or months, only to re-appear (or be increased) again. Usually, in RR-MS, the patient will go through- several consecutive cycles of such remittance and relapse, over a total period of time that may stretch from months to years.

In most patients, however, this RR-phase is eventually followed by a subsequent clinical phase, in which the paralysis phenomena no longer (completely) disappear, i.e. a clinical stage without (full) remittance. Thereafter, the patients usually show a steady - often essentially linear - increase in disease symptoms. This clinical phase is referred to as the secondary progressive (SP) phase.

Other, usually older, MS-patients do not first experience cycles of remittance and relapse (as in RR-MS), but show from the beginning a chronic progressive course of the disease. These patients are referred to as primary progressive (PP) patients.

Currently, these three clinical subtypes of MS are distinguished only on the basis of the symptoms shown by the patient, i.e. whether the patient experiences cycles of remittance/relapse, has a clinical history of remittance/relapse, and/or shows a progressive course of the disease. Unfortunately, this method may not be very accurate, as it relies in part upon the subjective experience of the patient. Also, it usually takes a certain period of time to establish whether there is (still) any remittance of symptoms (as in RR-MS) or not (as in SP-MS and PP-MS).

Furthermore, when the clinician relies only upon the occurrance of disease symptoms, it may not be possible to discriminate accurately - i.e. in all cases and/or with any degree of certainty - between secondary progressive and primary progressive patients. This is because the clinical history of the patient may not be (fully) known. Differences are likely due to differences in the pathogenesis of these subtypes.

Thus, there is a need for a method via which the different clinical subtypes of MS may be distinguished/determined. Such a method would not only make it possible to discriminate between RR-MS, SP-MS and PP-MS patients, but could also be used to monitor the progression of the disease, and in particular the transition from RR-MS to SP-MS, more accurately than by relying on disease symptoms alone. The invention provides such a method

This method could also be of significance because- as a result of the ongoing research into MS and the possible treatments thereof - it may prove that each of the different clinical subtypes of MS responds better to, or may even require, a different treatment or treatment regimen. For instance, IFN-β is often used in the treatment of RR-MS, but has not yet been fully proven to be effective in the treatment of SP-MS or PP-MS. Similarly, other medicaments could become available that show a higher succes rate against some clinical subtype(s) of the disease compared to others. This would make it very important for the clinician to be able to determine or further substantiate the clinical subtype of a patient quickly and accurately, and immediately upon the first onset of symptoms.

Furthermore, in both the study of the disease as well as in the development of possible treatments for MS, it would be very useful to be able to discriminate more accurately between the different clinical subtypes of MS. Again, such a method could be used to develop specific treatments of MS and/or to determine whether a proposed treatment is more successful against some subtype(s) of the disease compared to others. It is therefore envisaged that the method of the invention may not only have applications in clinical practice, but also in the field of MS research and/or drug development.

The invention is based upon the finding that the different clinical subtypes of MS can be discriminated by determining, in a biological sample and/or in a set of biological samples obtained from a patient, the level(s) of one or more specific cytokines. In particular, according to the invention, the level(s) of said one or more cytokines may be determined by measuring the amount(s) of the mRNA(s) encoding said cytokines in said biological sample(s), as will be further described hereinbelow.

Of particular interest in the invention are the levels of certain pro-inflammatory cytokines, and in particular of one or more of IL-18, IL-12p40, and IFN-γ; the levels of certain anti-inflammatory cytokines, and in particular of one or more of IL-4, IL-10 of TGF-β; as well as the level of IL-12 receptors (IL-12R), and in particular the level(s) of (mRNA encoding) the separate chains of the IL-12R, i.e. IL-12Rβ1 and/or IL-12Rβ2. Thus, according to the invention in its broadest sense, the level of at least one, and preferably of a combination of two or more, of these cytokines is determined in at least one biological sample obtained from a person suffering from - or suspected to suffer from - multiple sclerosis.

More in particular, in the research leading up to the present invention, it was found that, compared to healthy controls:
- RR-MS patients showed increased levels of IL-18, IL-12p40, TGF-β and IL-4;
- SP-MS patients showed increased levels of IL-18, TGF-β, IL-12p40 and IL-12Rβ2 and decreased levels of IL-10 and IFN-γ;
- PP-MS patients showed increased levels of IL-18 and IL-12Rβ2 and decreased levels of IL-10, IL-12Rβ1 and IFN-γ

It was also found that, compared to RR-MS patients:
- SP-MS patients showed increased levels of IL-12Rβ2 and IL-18 and decreased levels of IL-10, and IL12Rβ1. Also, it was surprisingly found that IFN-γ was strongly decreased;
- PP-MS patients showed increased levels of IL-12Rβ2 and decreased levels of IFN-γ, IL-12p40, IL-12Rβ1, IL-10, IL-4 and TGF-β.

Furthermore, it was found that, compared to SP-MS patients, PP-MS patients showed decreased levels of IL-12p40, IL-18, IL-12Rβ1, IL-12Rβ2 and TGF-β.

In terms of the specific cytokines involved, it was found that:
- IL-18 is increased in RR-MS and PP-MS patients compared to healthy individuals, and is increased even more in SP-MS patients. In PP-MS and RR-MS patients, IL-18 is about the same;
- IFN-γ is about the same in healthy individuals and RR-MS patients, but is decreased in PP-MS and SP-MS patients. In PP-MS and SP-MS patients, IFN-γ is about the same;
- IL-12p40 is increased in RR-MS and SP-MS patients compared to healthy individuals and PP-MS. In RR-MS and SP-MS patients, IL-12p40 is about the same;
- IL-12Rβ1 is about the same in healthy individuals, RR-MS patients and SP-MS patients, but decreased in PP-MS patients;
- IL-12Rβ2 is about the same in healthy individuals and in RR-MS patients, but is increased in PP-MS patients, and is increased even more in SP-MS patients;
- IL-10 is about the same in healthy individuals and in RR-MS patients, but is decreased in PP-MS and SP-MS patients. In PP-MS and SP-MS patients, IL-10 is about the same;
- IL-4 is increased in RR-MS patients compared to healthy individuals and PP- patients
- TGF-β is increased in RR-MS patients compared to healthy individuals and PP-MS, and increased even more in SP-MS patients. In PP-MS, TGF-β is about the same as in healthy controls.

By contrast, TNF-α and IL-12p35 are about the same in healthy individuals, RR-MS patients, SP-MS patients and PP-MS patients.

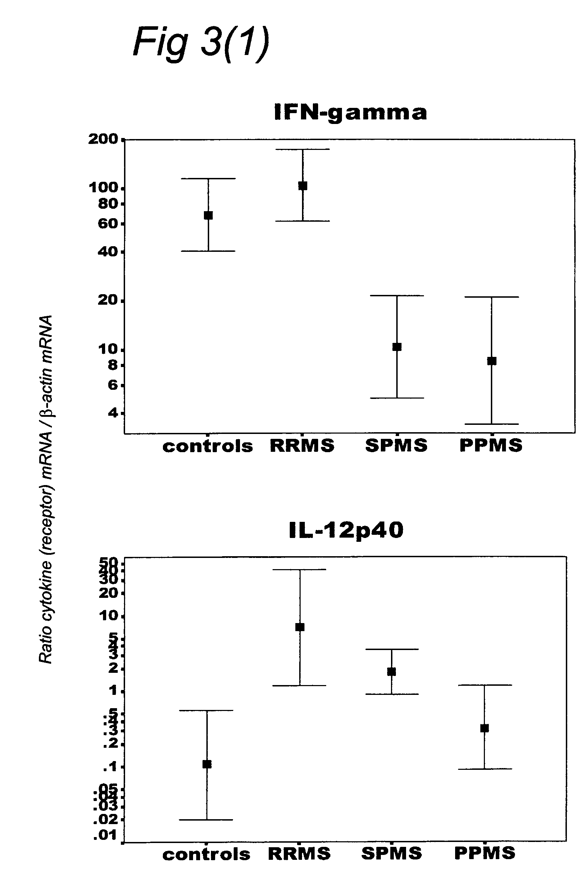

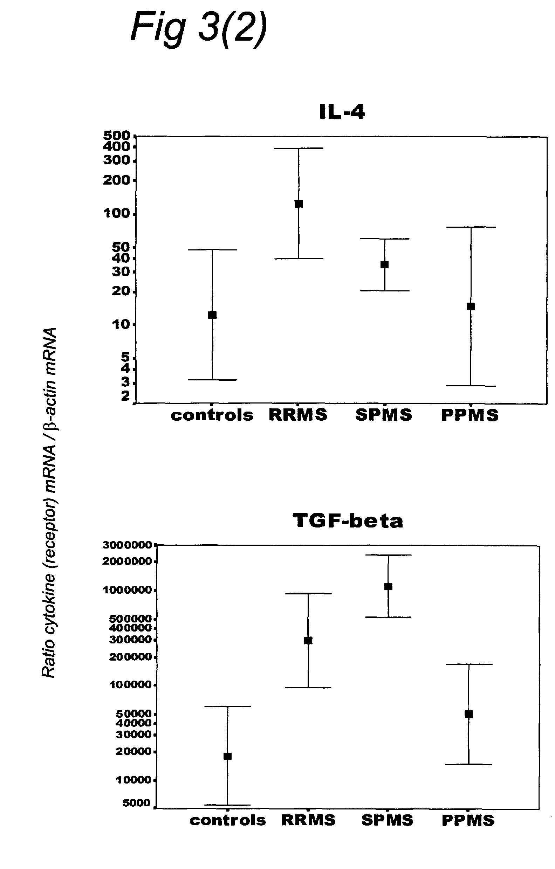

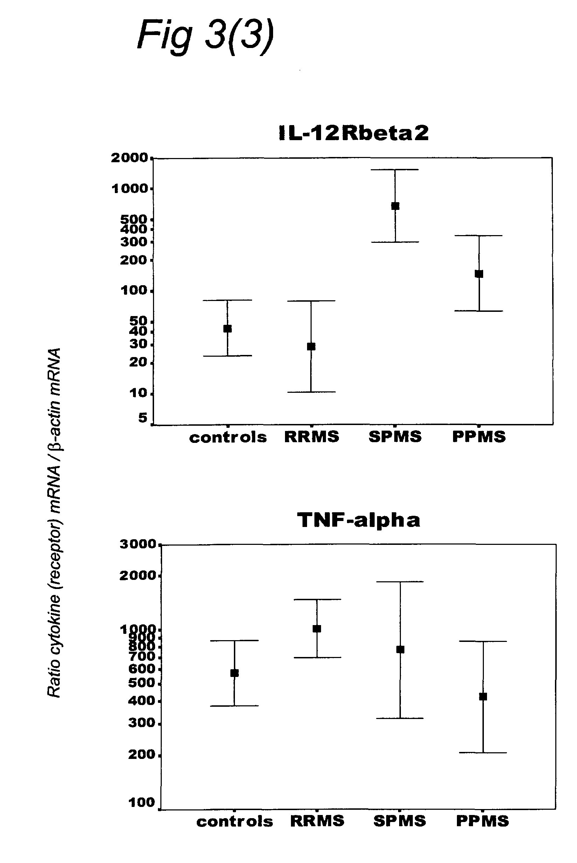

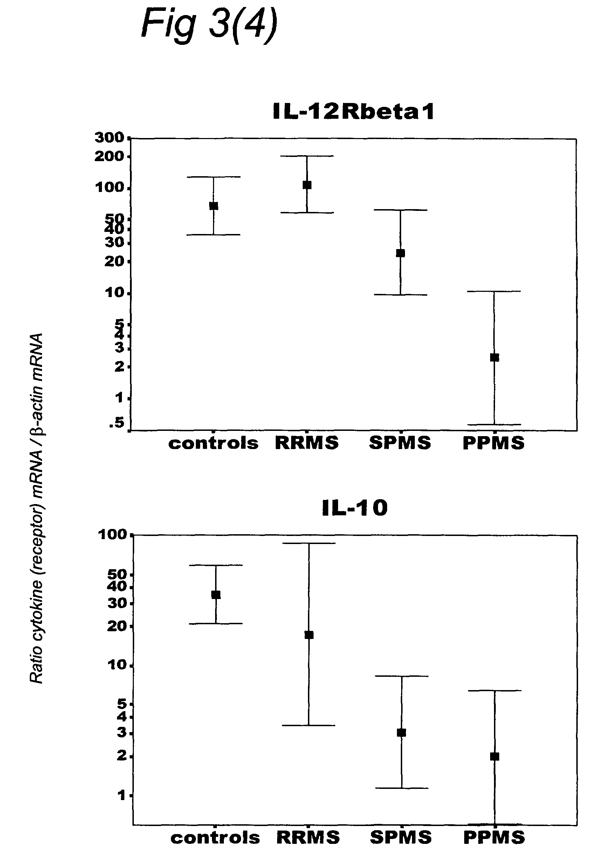

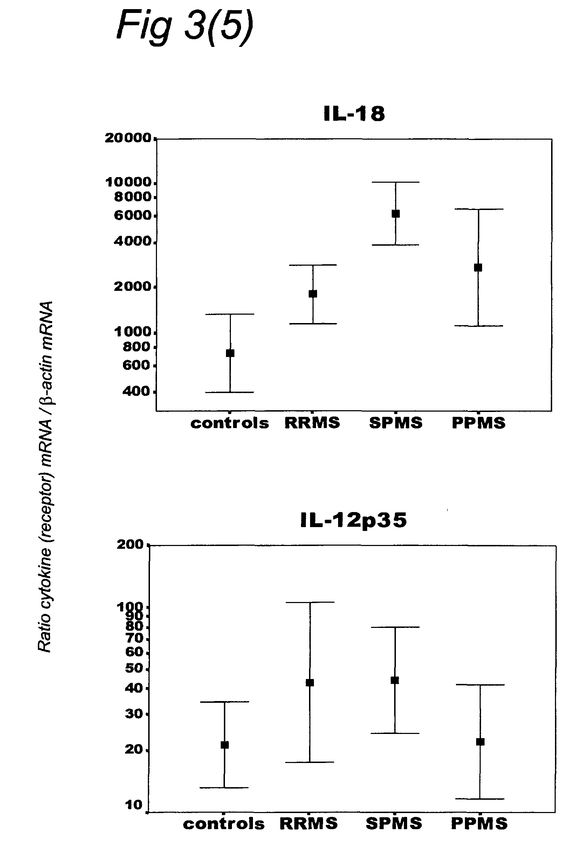

It should be noted that in this context, when it is stated that the values/levels for certain cytokines determined in or for certain clinical subtypes are "about the same", it is meant that the values/levels obtained for said cytokines are not sufficiently different - e.g. in absolute terms and/or statistically - to allow a distinction to be made between the different subtypes of the disease based upon this specific cytokine (alone). It does not necessarily mean that the values/levels measured for said cytokine for each subtype of the disease are about equal in absolute terms (although this will usually be the case). Reference is made to the Table 6 and the Figures 3-5.

It will be clear to the skilled person that the above enables the skilled person to discriminate between healthy individuals, RR-MS patients, SP-MS patients and PP-MS patients, i.e. by measuring one or more of the cytokines mentioned above.

For instance, RR-MS and SP-MS can be distinguished by means of the levels of one or more of IL-18, IFN-γ, IL-10, IL-12Rβ1 and/or IL-12Rβ2, and is preferably distinguished by the levels of IFN-γ, IL-10 and IL-12Rβ2.

PP-MS and SP-MS can be distinguished by means of the levels of one or more of IL-12p40, TGF-β, IL-18, IL-12Rβ1 and/or IL-12Rβ2, and is preferably distinguished by the levels of TGF-β.

Furthermore, although possibly less relevant in practice, RR-MS and PP-MS can be distinguished by means of the levels of IFN-γ, IL-12p40, IL-4, IL-10, TGF-β, IL-12Rβ1 and/or IL-12Rβ2, and is preferably distinguished by the levels of IFN-γ, IL-12Rβ1 and IL-12Rβ2.

Also, it will be clear to the skilled person that the level(s) of TGF-β, IL-12Rβ1 and/or IL-12Rβ2 and/or IFN-γ may be used to discriminate between all three of RR-MS, SP-MS and PP-MS, optionally in combination with one or more of the other cytokines mentioned above.

Thus, in a first aspect, the invention relates to a method for determining the success rate of treatment of multiple sclerosis by discriminating between patients with multiple sclerosis (regardless of the clinical subtype) and healthy controls, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of IFN-γ, IL-12p40, IL-12Rβ1, IL-12Rβ2 and IL-18 mRNA is determined, optionally in combination with at least one, or possibly at least two of the other cytokines mentioned above. Even more preferably, in this aspect of the invention, in step b) at least IL-18 is determined, optionally in combination with at least one, or possibly at least two of the other cytokines mentioned above.

In a second aspect, the invention relates to a method for determining the success rate of treatment of multiple sclerosis by discriminating between the different subtypes of multiple sclerosis, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of TGF-β, IFN-γ, IL-10, IL-12Rβ1 and/or IL-12Rβ2 is determined, optionally in combination with at least one, or possibly at least two of the other cytokines mentioned above.

Even more preferably, in this aspect of the invention, in step b), at least one or more of the group consisting of TGF-β, IFN-γ, and/or IL-12Rβ2 is determined, optionally in combination with at least one, or possibly at least two of the other cytokines mentioned above.

In a third aspect, the invention relates to a method for determining the success rate of treatment of multiple sclerosis by discriminating between relapsing remitting multiple sclerosis and secondary progressive multiple sclerosis, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IL-18, IFN-γ, IL-10, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b), one or more of the group consisting of IL-12Rβ2, IFN-γ and IL-10 is determined, optionally in combination with at least one, or possibly at least two of the other cytokines mentioned above. Even more preferably, in this aspect of the invention, in step b) at least IL-12Rβ2 is determined, optionally in combination with at least one, or possibly at least two of the other cytokines mentioned above.

In a fourth aspect, the invention relates to a method for determining the success rate of treatment of multiple sclerosis by discriminating between primary progressive multiple sclerosis and secondary progressive multiple sclerosis, on the basis of at least one biological sample obtained from a person suffering from or suspected to suffer from multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IL-12p40, IL-18, TGF-β, IL-12Rβ1 and/or IL-12Rβ2; and optionally
c) comparing, for one or more of the cytokines determined in step b), the value obtained in step b) with at least one reference value, e.g. for the same cytokine.

Preferably, in this aspect of the invention, in step b) one or more of the group consisting of TGF-β, IL-12Rβ1 and IL-12Rβ2 is determined, optionally in combination with at least one, or possibly at least two of the other cytokines mentioned above. Even more preferably, in this aspect of the invention, in step b) at least TGF-β is determined, optionally in combination with at least one, or possibly at least two of the other cytokines mentioned above.

In a further aspect, the invention relates to a method for discriminating between remitting relapsing multiple sclerosis and primary progressive multiple sclerosis, on the basis of at least one biological sample obtained from a person suffering from or suspected to suffer from multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IFN-γ, IL-12p40, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of IL-12Rβ1, IL-12Rβ2 and IFN-γ is determined, optionally in combination with at least one, or possibly at least two of the other cytokines mentioned above. Even more preferably, in this aspect of the invention, in step b) at least IFN-γ is determined, optionally in combination with at least one, or possibly at least two of the other cytokines mentioned above.

Also, the invention relates to a method for determining the success rate of treatment of multiple sclerosis by determining whether a biological sample (or a set of biological samples) has been derived from a patient with multiple sclerosis (regardless of the clinical subtype) or from a healthy control, said method generally comprising the steps of:
a) providing said biological sample(s) from a patient suffering from, or suspected to suffer from, multiple sclerosis;
b) determining in said biological sample(s) the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of IFN-γ, IL-12p40, IL-12Rβ1, IL-12Rβ2 and IL-18 mRNA is determined, optionally in combination with at least one, or possibly at least two of the other cytokines mentioned above. Even more preferably, in this aspect of the invention, in step b) at least IL-18 is determined, optionally in combination with at least one, or possibly at least two of the other cytokines mentioned above.

Also, the invention relates to a method for determining the success rate of treatment of multiple sclerosis by determining whether a biological sample (or a set of biological samples) has been derived from a patient with multiple sclerosis, and if so, whether said (set of) biological sample(s) has been derived from a patient suffering from relapsing remitting multiple sclerosis, secondary progressive multiple sclerosis and/or primary progressive multiple sclerosis, said method generally comprising the steps of:
a) providing said biological sample(s) from a patient suffering from, or suspected to suffer from, multiple sclerosis;
b) determining in said biological sample(s) the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of TGF-β, IFN-γ, IL-10, IL-12Rβ1 and/or IL-12Rβ2 are determined, optionally in combination with at least one, or possibly at least two of the other cytokines mentioned above.

Even more preferably, in this aspect of the invention, in step b), at least one or more of the group consisting of TGF-β, IFN-γ, and/or IL-12Rβ2 is determined, optionally in combination with at least one, or possibly at least two of the other cytokines mentioned above.

The invention further relates to a method for determining the success rate of treatment of multiple sclerosis by determining whether a biological sample (or a set of biological samples) has been derived from a patient with relapsing remitting multiple sclerosis or from a patient with secondary progressive multiple sclerosis, said method generally comprising the steps of:
a) providing said biological sample(s) from a patient suffering from, or suspected to suffer from, multiple sclerosis, and in particular from a patient suspected to suffer from either relapsing remitting multiple sclerosis or secondary progressive multiple sclerosis;
b) determining in said biological sample(s) the amount of at least one of the cytokines from the group consisting IL-18, IFN-γ, IL-10, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of IL-12Rβ2, IFN-γ and IL-10 is determined, optionally in combination with at least one, or possibly at least two of the other cytokines mentioned above. Even more preferably, in this aspect of the invention, in step b) at least IL-12Rβ2 is determined, optionally in combination with at least one, or possibly at least two of the other cytokines mentioned above.

The invention further relates to a method for determining the success rate of treatment of multiple sclerosis by determining whether a biological sample (or a set of biological samples) has been derived from a patient with primary progressive multiple sclerosis or from 5 a patient with secondary progressive multiple sclerosis, said method generally comprising the steps of:
a) providing said biological sample(s) from a patient suffering from, or suspected to suffer from, multiple sclerosis, and in particular from a patient suspected to suffer from either primary progressive multiple sclerosis or secondary progressive multiple sclerosis;
0 b) determining in said biological sample(s) the amount of at least one of the cytokines from the group consisting of IL-12p40, IL-18, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of TGF-β, IL-12Rβ1 and IL-12Rβ2 is determined, optionally in combination with at least one, or possibly at least two of the other cytokines mentioned above. Even more preferably, in this aspect of the invention, in step b) at least TGF-β is determined, optionally in combination with at least one, or possibly at least two of the other cytokines mentioned above.

The invention further relates to a method for determining the success rate of treatment of multiple sclerosis by determining whether a biological sample (or a set of biological samples) has been derived from a patient with relapsing remitting multiple sclerosis or from a patient with primary progressive multiple sclerosis, said method generally comprising the steps of:
a) providing said biological sample(s) from a patient suffering from, or suspected to suffer from, multiple sclerosis, and in particular from a patient suspected to suffer from either relapsing remitting multiple sclerosis or primary progressive multiple sclerosis
b) determining in said biological sample(s) the amount of at least one of the cytokines from 5 the group consisting of IFN-γ, IL-12p40, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

Preferably, in this aspect of the invention, in step b) one or more of the group consisting of IL-12Rβ1, IL-12Rβ2 and IFN-γ is determined, optionally in combination with at least one, or possibly at least two of the other cytokines mentioned above. Even more preferably, in this aspect of the invention, in step b) at least IFN-γ is determined, optionally in combination with at least one, or possibly at least two of the other cytokines mentioned above.

Also, according to this aspect of invention, it is possible to discriminate first between 5 only one of the three subtypes on the one hand and the other two on the other hand (e.g. between RR-MS on the one hand and SP-MS/PP-MS on the other; or between PP-MS and RR-MS/SP-MS), and then, if still required, to distinguish between the two remaining subtypes.

However, by determining at least TGF-β, IFN-γ, IL-10, IL-12Rβ1 and IL-12Rβ2; or by 0 determing at least IFN-γ, TGF-β and IL-12Rβ2; optionally in combination with at least one of the other cytokines mentioned above, it is possible to discriminate between all three subtypes simultaneously, e.g. on a single sample or a single set of samples.

Also, according to a similar aspect of the invention, one or more of the cytokines of the group consisting of IL-18, IFN-γ, TGF-β and/or IL12Rβ2 may be determined, optionally in combination with at least one of the other cytokines mentioned above.

In order to determine whether the level of a certain cytokine is increased or decreased, the values measured for a patient can optionally be compared with one or more reference values for said cytokine, e.g. values that can be considered representative for the level(s) of said specific cytokine in healthy individuals and/or for each of the subtypes RR-MS, SP-MS and PP-MS.

For instance, the reference value(s) may be the mean value(s) obtained of the pertinent comparable samples from a representative group of healthy persons, RR-MS patients, SP-MS patients and/or PP-MS patients, determined under identical conditions as the sample(s) and assay conditions regarding the (suspected) MS patient(s). The reference value(s) may also for instance be generated from clinical experience and/or from a statistical analysis of earlier results. Also, it is envisaged that - based upon the disclosure herein - such values may become publicly available, either in the scientific literature or as part of a database.

The reference value(s) may also be generated by measuring one or more reference sample(s) that can be considered representative for healthy individuals, RR-MS patients, SP-MS patients and/or PP-MS patients, respectively. In particular, such reference values may be generated essentially simultaneously with the measurement of the one or more sample(s) to be determined, e.g. as part of a single set of measurements/assays. This allows direct comparison of the results obtained and thus may be preferred, as the measurement of absolute numbers of the above cytokines may be subject to variation due to for instance the type of assay used and the precise assay conditions.

Another possibility is to measure essentially simultaneously - e.g. as part of a single set 5 of measurements/assays - the sample and cytokine(s) of interest in relation to an external standard, of which the value it is supposed to give in the particular assay is known, and compare thus obtained related value to the corresponding related reference value. Also in this case care must be taken that assay and further assay conditions and circumstances are essentially identical.

Furthermore, when the method of the invention is used to follow the progression of the disease - in particular the transition of RR-MS to SP-MS - it may be possible to use as the reference value(s) value(s) that have been measured earlier for samples derived from the same patient. A change in the value(s) for one or more of the cytokines of interest - for instance, in case of the transition from RR-MS to SP-MS, one or more of IL-18, IFN-γ, IL-10, IL12Rβ1 and/or IL-12Rβ2 - could then be indicative of a transition from one subtype to another.

Usually, in the practice of the invention, the values for one or more individual cytokines or for several cytokines will be compared with the reference values for the same cytokines in healthy individuals and/or in one or more - and preferably all - of the other subtypes. Also, the pattern of differences/changes in the values for a number of cytokines may be compared to the pattern for corresponding set of cytokines in healthy individuals and/or in one or more - and preferably all - of the other subtypes.

In doing so, the values measured for a sample derived from a patient with a specific subtype will generally provide, for the cytokine(s) of interest, essentially the same values or pattern of values as the corresponding reference value(s) that are representative for the same subtype; and/or will provide the same differences or pattern of differences compared to the reference value(s) that are representative for healthy individuals and/or that are representative for the other two subtypes, i.e. as outlined hereinabove (although of course some individual variation from patient to patient cannot be excluded).

Thus, for example, when for the cytokine(s) of interest, a sample obtained from a patient shows essentially the same value(s) or essentially the same pattern of values as the reference value(s) representative for SP-MS; and/or shows one or more of the difference(s) in values or in pattern of values described hereinabove ( i.e. compared to the reference values representative for healthy individuals and/or for RR-MS or PP-MS patients); and/or essentially shows one or more of the difference(s) in values or in pattern of values that a reference sample for SP-MS also shows (i.e. compared to the reference samples that are representative for healthy individuals and/or for RR-MS or PP-MS patients), the patient may be classified as a SP-MS patient. Of course, the degree of certainty that can be attributed to said classification will usually increase with the total number of different cytokines measured, with the total number of values measured that correspond to the values that are representative for a certain subtype and/or that differ from the values that are representative for healthy individuals and for the other subtypes; as well as with a higher degree of conformity with the pattern of values expected for a specific subtype (and less conformity with the pattern of values expected for the other subtypes).

Thus, on the basis of the disclosure herein, optionally in combination with a limited degree of (clinical) experience that can easily be obtained in the manner described herein, the skilled person will be able to discriminate, in or for individual cases, between the three subtypes of MS.

As such, the classification obtained for an individual patient according to the above described aspects of the invention, besides allowing the determination of the success rate of treatment of MS with IFN-β, may (also) be of some help to the clinician in determining the manner and course of any (further) treatment of the patient. Also, the method of the invention may be of use in assessing and/or comparing, for each of the subtypes of MS, the effectiveness of available or proposed methods, regimens, active substances, pharmaceutical compositions, etc. for the treatment of the disease. These include both treatments that are available or have been proposed at the date of filing of this application, as well as such treatments that may become available or that may be proposed in future.

The level/amount of each cytokine of interest is preferably determined by measuring the amount of mRNA encoding said cytokine that is present in the biological sample. However, the invention is not limited thereto, and any method for determining the amount of said cytokines, such as for instance flow cytometry or ELISA based methods using for instance commercially available reagents, in a biological sample will fall under the scope of the invention. For instance, it is also possible to measure one or more of the cytokines at the protein level, e.g. using a suitable technique such as ELISA-techniques for protein measurement. Also, with respect to TGF-β, usually the amount of TGF-β1 will be measured, although the invention in its broadest sense is not limited thereto, and also for instance other TGF-β subtypes and/or essentially all TGF-β subtypes simultaneously may be measured.

At present, in general, patients suffering from multiple sclerosis (MS) are treated with interferon, in particular Interferon-beta (IFN-β) species such as IFN-β1b and IFN-β1a. Several forms of MS exist, such as primary progressive (PP), secondary progressive- (SP) and relapsing remitting (RR) multiple sclerosis, and in particular the latter (RR MS) is treated with IFN-β. This treatment inhibits progress of the disease by roughly one-third. The mechanism of action of IFN-β is, at present, unclear (for a review see: Yong et al., Neurology 1998;51:682-689).

Unfortunately, however, treatment with IFN-β is not effective in all patients. An estimate is that more than one third (38.5 %) of the MS patients that undergo IFN-β treatment do not benefit from this. Not only is the treatment with IFN-β a costly one, but also puts a strain on patients living in uncertainty whether they will profit from the treatment or not. Therefore it is desirable to be able to discriminate prior to, or at least shortly after treatment has started, between patients that will benefit from treatment with IFN-β and those that will not.

It has now been found that such a discrimination can be made by determining the amount (of mRNA encoding) of at least one, preferably more, of the cytokines mentioned below present in a biological sample obtained from a patient. More specifically, it has been found that information on the chances of success of a therapy with IFN-β can be obtained by comparing levels of certain mRNA's encoding certain cytokines between blood samples collected before the start of IFN-β treatment and after IFN-β treatment has been initiated, i.e. upon treatment for a period of 1 to 20 days, or 20 to 40 days, or 40 to 60 days, or 60 to 80 days, preferably for a period of 20 to 40 days, more preferably for 30 days.

Since long it is assumed that many cytokines, such as interleukin-12 (IL-12), tumour-necrosis-factor-alpha (TNF-α), transforming-growth-factor-beta (TGF-β), IFN-γ, IL 10 and IL-4, play a role in multiple sclerosis (for a review see Navikas and Link, J. Neurosci. Res. 1996;45:322-333). However the exact role of each cytokine in the mechanism of multiple sclerosis is largely, if not completely, unknown. In particular, nothing is known in the art about the predictive value of the values for (the mRNA's encoding) each of the cytokines in predicting/assessing the chance of success of MS therapy. The underlying patterns in the mRNA levels of the cytokines as disclosed in the subject invention and the consequences for the success of IFN-β treatment thereof are therefore unexpected and provide a surprisingly efficient tool for determining the success rate of treatment of multiple sclerosis.

In a further aspect, the method according to the invention comprises the steps of:
a) providing a biological sample derived from a patient suffering from, or suspected to suffer from, multiple sclerosis;
b) determining the amount of at least one of the cytokines from the group consisting of IL-12p35, IL-18 and TGF-β of said first biological sample;
c) optionally comparing the value obtained in step b) with a reference value.

According to this aspect of the invention, either the amount of IL-12p35, of IL-18 and/or of TGF-β, the amount of any two thereof, and/or the amount of all three thereof may be determined, e.g. prior to start of a (contemplated) treatment with IFN-β. If two or more of IL-12p35, IL-18 and TGF-β are determined, this may be done in/from the same sample or in/from (a set of) equivalent samples, e.g. obtained essentially simultaneously from the patient. Preferably in step b) at least the amount of (mRNA encoding) IL-12p35 is determined.

The value(s) thus obtained can be compared with the corresponding reference value(s), for instance with the corresponding values from comparable samples obtained from healthy individuals. For instance, such a reference value may be the mean value obtained from comparable samples from a representative group of healthy persons, determined under identical conditions as the sample(s) and assay conditions regarding (suspected) MS patients. The measurement of absolute numbers of these cytokines is subject to variation due to for instance the type of assay used and the precise assay conditions. Therefore it is preferred to simultaneously measure, in one assay, one or more reference sample(s) and one or more sample(s) of (suspected) MS patients. This allows direct comparison of the results. Another possibility is to measure in one assay the sample and cytokine of interest in relation to an external standard, of which the value it is supposed to give in the particular assay is known, and compare thus obtained related value to the corresponding related reference value. Also in this case care must be taken that assay and further assay conditions and circumstances are essentially identical.

According to the invention, it has been found that, by determining the mRNA encoding for IL-12p35,IL-18 or TGF-β in patients before they undergo treatment with IFN-β, already a fair chance of responsiveness for IFN-β treatment can be given. For instance, based upon the amount of IL-12p35 mRNA in a sample obtained from a patient before (a contemplated) treatment is initiated, the success of said treatment may already be correctly predicted in at least 75%, up to essentially 80%, of the cases. For IL-18 and TGF-β these percentages amount to at least 50% and at least 60%, respectively.

In a further aspect, the method according to the invention comprises the steps of:
a) providing a first biological sample derived from a patient suffering from, or suspected to suffer from, multiple sclerosis;
b) determining at least the amount of IL-10 of said first biological sample
c) providing a second biological sample from the patient of step a), after said patient has been treated with interferon-β for a period of 1 to 20 days, or 20 to 40 days, or 40 to 60 days, or 60 to 80 days, preferably for a period of 20 to 40 days, more preferably for 30 days;
d) determining at least the amount of IL-10 of said second biological sample;
e) optionally comparing the value measured during step b) with the value measured during step d).

According to this aspect of the invention, in step b) the amount of (mRNA encoding) interleukin-10 is determined before treatment is initiated. In step d) the amount of (mRNA encoding) interleukin-10 is again determined after treatment has been initiated, i.e. after one of the above indicated time periods, preferably for instance after approximately 1 month of IFN-β treatment, and this value is then compared to the value obtained in step b). If the value for (mRNA encoding) IL-10 after said preferred period of treatment - i.e. the value measured in step d) - is at least 2-fold higher compared to the value measured prior to treatment - i.e in step b) - it may be assumed, with greater reliability (infra), that further treatment with IFN-β will not be successful. However, if the value for (mRNA encoding) IL-10 in step d) is less than a factor 2 higher it may be assumed, with greater reliability (infra), that further treatment with IFN-β will be successful

Optionally, further valuable information can be obtained by carrying out both the latter two methods mentioned above, and then assessing the results/information obtained from said methods in conjunction with each other.

In particular, the results from step b) of the second last method mentioned above for IL-12p35, IL-18 or TGF-β, or at least two, optionally all three of these compounds, before treatment, may be assessed in conjunction with the data for IL-10 before treatment (i.e. from step b) of the last method hereinabove) and after a period of 1 to 20 days, or 20 to 40 days, or 40 to 60 days, or 60 to 80 days, preferably after 20 to 40 days, more preferably after 30 days (step d from the second method hereinabove) of treatment with IFN-β. Such a method combining both the the latter two methods mentioned above may therefore comprise the steps of:
a) providing a first set of one or more biological sample(s) derived from a patient suffering from, or suspected to suffer from, multiple sclerosis;
b) determining at least the amount of IL-10 in at least one sample from the first set obtained in step a); and also determining the amount of at least one of the cytokines chosen from the group IL-12p35, IL-18 and TGF-β in the same or a different sample from the first set obtained in step a);
c) optionally comparing the value obtained in step b) for IL-12p35, IL-18 and TGF-β with a reference value;
d) providing a second set of one or more biological sample(s) from the patient of step a), after said patient has been treated with interferon-(β for a period of 1 to 20 days, or 20 to 40 days, or 40 to 60 days, or 60 to 80 days, preferably for a period of 20 to 40 days, more preferably for 30 days;e) determining at least the amount of IL-10 in at least one sample of the second set obtained in d);
f) optionally comparing the value(s) measured for IL 10 during step b) with the value(s) measured during step e).

According to this "combined method" of the invention, in step b), either the amount of IL-12p35, the amount of IL-18 and/or the amount of TGF-β may be determined, any two thereof, or all three thereof, i.e. in the same or in different samples of the first set of samples obtained in step a), which sample(s) may also be the same or different from the sample used for determining the amount of IL-10. Again, these values may be determined in/from a single sample or in/from different samples of the second set of samples obtained in step d), which sample(s) may also be the same or different from the sample used for determining the amount of IL-10 in step e).

The values thus obtained for IL-12p35, IL-18 or TGF-β after treatment has been initiated may then be assessed in conjunction with each other and/or with any or all of the other values obtained from said combined method, e.g those for IL-12p35, IL-18 or TGF-β before treatment was initiated, as well as those for IL-10. This may provide additional valuable information on the (expected) effectiveness of treatment.

Preferably, in this combined method, besides the amount of (mRNA encoding) IL-10, the amount of at least (mRNA encoding) IL-12p35 is determined. By doing so the reliability of the prediction may be increased. For instance, determining the amount of IL-10 mRNA before treatment and after one of the above indicated periods of time of treatment with IFN-β, and assessing the data thus obtained in conjunction with the data on the amount IL-12p35 mRNA determined before treatment, and optionally after one of the above indicated periods of time of treatment, may result in a prediction of the success of therapy with IFN-β which may have a reliability of more than 90% of all cases, and up to essentially 100%. A related parameter to determine if a patient could benefit from IFN-β treatment would be for instance the ratio of IL-12p35 mRNA to IL-10 mRNA before the start of treatment and after one of the above indicated periods of time, preferably after approximately 1 month, of treatment. In patients that benefit from treatment this ratio will usually only be slightly affected, whereas in patients that do not benefit from treatment this ratio will usually strongly decrease, e.g. according to the values set out below.

Thus, according to the invention the amount of the cytokines IL-12p35, IL-18, TGF-β and/or IL-10 in one or more biological samples from a patient is determined. This is preferably achieved by determining the amount of mRNA encoding these cytokines present in the biological sample. However, the invention is not limited thereto, and any method for determining the amount of said cytokines, such as for instance flowcytometry or ELISA based methods using for instance commercially available reagents, in a biological sample will fall under the scope of the invention. More specific, the amount of a particular cytokine in a biological sample can also be determined as the amount of protein present in said sample. Especially for differentiation of cytokines at a cellular level, intracellular staining techniques are feasible. Kits for staining particular cytokines are commercially available.

Improvement of the reliability of determining the success rate of treatment with IFN-β can be achieved by studying the accumulation of IL-10 at the protein level. Prior to treatment both responders and non-responders to IFN-β therapy - as compared to healthy controls-show decreased numbers of T cells, in particular CD4⁺ T cells, that are capable to accumulate IL-10 without additional in vitro stimulation. A comparison of peripheral blood mononuclear cells from a clinical responder obtained before treatment, with a sample obtained 3 or 6 months after start of treatment shows a significant increase in the number of such cells. This does not happen in the patients that have no clinical benefit from therapy.

IFN-β treatment simultaneously is associated with a downregulation of IL-10 in monocytes. This happens in responders as well in non-responders. Thus, treatment deactivates monocytes in responders and non-responders and stimulates IL-10 in the T cells from responders only. Consequently, changes at the protein level do not necessarily have to match changes at the mRNA level. This is in line with the amounts of mRNA found for IL-10 in responders and non- responders as described above.

Thus in a further aspect the method according to the invention comprises the steps of:
a) providing a first biological sample derived from a patient suffering from, or suspected to suffer from, multiple sclerosis;
b) determining at least the amount of IL-10 protein in CD4⁺ T cells of said first biological sample;
c) providing a second biological sample from the patient of step a), after said patient has been treated with interferon-β for a period of 0 to 50 days, or 50 to 100 days, or 100 to 150 days, or 150 to 200 days, preferably for a period of 150 to 200 days, more preferably for 180 days;
d) determining at least the amount of IL-10 protein in CD4⁺ T cells of second biological sample;
e) optionally comparing the value measured during step b) with the value measured during step d).

In addition, the determination of IL-10 protein in CD4⁺ T cells can be used in combination with other determinations of IL-10, IL-12p35, IL-18 and/or TGF-β, hereinabove referred to as the "combined method" of the invention. Combining the information obtained on IL-10 in CD4⁺ T in a similar fashion as described for the combined method incrases the reliability of determining the success rate of treatment.

Also according to the invention any of the methods for determing the success rate of treatment of multiple sclerosis by discriminating between patients with multiple sclerosis can be combined with any of the methods for determing the success rate of treatment of multiple sclerosis in which IL-10, IL-12p35, IL-18 and/or TGF-β and/or IL-10 protein in CD4⁺ T cells are determined. This constitutes an even further improvement of the reliability of determining the success rate of treatment with IFN-β. This method according to the invention allows determination of the subtype of MS that a patient is suffering from in conjunction with the (expected) effectiveness of treatment with IFN-β. On the basis of the information disclosed herein the skilled person will be able select one or more combinations of pertinent cytokines selected from the group consisting of IL-18, IL-12p40, IFN-γ, IL-4, IL-10, TGF-β, IL-12Rβ1, IL-12Rβ2 and/or IL-12p35 which will allow determination of the success rate of treatment with IFN-β.

The amount of mRNA encoding the pertinent cytokine can be determined in any biological sample obtained from a patient that contains such a cytokine, and preferably (expressed) mRNA encoding said cytokine. Preferably, the amount of mRNA is determined in a blood sample from the patient. Isolation of mRNA and measurement of specific mRNA from blood samples is carried out by procedures available to one skilled in the art. Standard mRNA isolation kits to obtain mRNA form lysed blood samples are commercially available. To be able to detect mRNA this can for instance be routinely multiplied using the reverse transcriptase - polymerase chain reaction tandem.

The cDNA obtained from the reverse transcriptase step is then quantified, for instance by comparison with an external standard that is amplified simultaneously, i.e. as part of the same set of amplification reactions and/or under essentially the same conditions. For this purpose any suitable amplification standard known per se, e.g. developed from stimulated or unstimulated lymphocytes, is effective. Another option might be the use of an internal standard of which a known amount is added to sample in order to become amplified simultaneously, provided that this standard gives rise to a product (preferably of known size) that differs in size from the product generated from the unknown sample.

For the amplification of the pertinent mRNA's encoding the cytokines, such as IL-12p35 mRNA and IL-10 mRNA, any suitable primer or set of primers can be used that can hybridize with the pertinent mRNA so as to allow amplification, i.e. under the conditions used for the amplification reaction, which will usually be conditions known per se such as those described by Visser et al., Blood 1998;91:4255-4264; and by van Boxel-Dezaire et al., Ann. Neurol. 1999;45:695-703.

Suitable primers will usually have a size of 18 to 25 nucleotides, and can be chosen by the skilled person on the basis of the sequence of the pertinent mRNA or one or more suitable parts thereof, which if necessary can be determined using known sequencing techniques. The primers are most preferably chosen such that they are selective for the intended cytokine mRNA (i.e. in that - from all mRNAs present in the biological sample - only the mRNA that encodes the cytokine to be determined is amplified) and also preferably chosen such that-upon amplification - amplified fragments are obtained that can be detected and quantitatively determined using known techniques for detecting/measuring amplified nucleic acid sequences, such as those described in the Experimental Part below. Appropriate selection of the primer(s) will be within the skill of the artisan.

Some preferred, but non-limiting examples of suitable primers include the primers disclosed by Visser et al., Blood 1998;91:4255-4264; and by van Boxel-Dezaire et al., Ann. Neurol. 1999;45:695-703, which are as follows:
IL-12p35 sense: CAGCAACATGCTCCAGAAGG
IL-12p35 antisense: CCTAGTTCTTAATCCACATC
IL-10 sense: ATGCTTCGAGATCTCCGAGA
IL-10 antisense: AAATCGATGACAGCGCCGTA
IFN-γ sense: GCAGAGCCAAATTGTCTCCT
IFN-γ antisense: ATGCTCTTCGACCTCGAAAC
IL-12p40 sense: GGAGTACTCCACATTCCTAC
IL-12p40 antisense: CCATGGCAACTTGAGAGCTG

For the amplification of IL-18, IL-4 and TGF-β, for instance the following primers can be used:
TGF-β sense: AGCGACTCGCCAGAGTGGTTATCTT
TGF-β antisense: TTATGCTGGTTGTACAGGGCCAGGA
IL-18 sense: GCTTGAATCTAAATTATCAGTC
IL-18 antisense: GAAGATTCAAATTGCATCTTAT
IL-4 sense: TGCCTCCAAGAACACAACTG
IL-4 antisense: AACGTACTCTGGTTGGCTTC

For the amplification of IL-12Rβ1 and IL-12Rβ2, for instance the following primers can be used:
IL-12Rβ1 sense: CCTGAAAACCCCCCACAG
IL-12Rβ1 antisense: GCTGAGGCATTGCCCCCA
IL-12Rβ2 sense: CTGCAAACTGGCCTGTATCAA
IL-12Rβ2 antisense: GTGCTCTCAATGATTCACTCC

Following the amplification step, the PCR products can be quantified in a manner known per se. For instance realtime PCR is a method for determining the amount of PCR product. Another method could be for instance separation on agarose followed by visualisation and quantification employing for instance radioactive methods or densitometric imaging. The values thus obtained may be normalised, for instance vis a vis β-actin standard equivalents or any other suitable gene standard.

Thus, in a preferred embodiment according to the invention, the amount of said cytokines is determined by isolating mRNA from a blood sample, transcribing the isolated mRNA into cDNA using reverse transcriptase, amplifying the cDNA product using the polymerase chain reaction employing appropriate primers, separating the PCR products on a gel and quantifying the separated products.

If in any method or aspect of this invention herinabove, the level of two or more cytokines is to be determined, this may be carried out in/on a single sample and/or on two or more separate samples from a set of essentially equivalent biological samples. Also, the measuring of each of the cytokines may be carried out essentially simultaneously and/or consecutively.

By a "set of samples" or a "set of essentially equivalent biological samples" (i.e. of first or second samples) is generally meant a collection of two or more biological samples that are essentially the same/equivalent in that they have been obtained from the patient essentially simultaneously and/or at essentially the same stage of the disease or treatment (if any). For instance, such a set of samples may comprise two or more blood samples that have been obtained from the patient on the same day(s) or at least within a few days from each other.

Usually, the samples in such a set will all be comprised of the same biological material or fluid - for example, a set of blood samples - although the invention is not limited thereto.

The biological sample may be any suitable biological material or fluid obtained from the patient (including but not limited to samples obtained through biopsy), but is most preferably blood or a blood sample.

In one non-limiting embodiment of the invention, each of the cytokines to be determined is measured separately, i.e. in a separate assay, for instance in separate sample(s) from a set of essentially equivalent biological samples and/or by dividing the at least one biological sample obtained from the patient into several portions and then measuring each of the cytokines to be determined in a different portion.

As such, the sample may be whole blood or a specific part, fraction or preparation thereof, such as fraction containing a specific cell population. For instance, a blood fraction or blood preparation containing (a population of) peripheral blood mononuclear cells may be used, such as a fraction containing (a population of) leukocytes. Such a fraction may for instance be obtained starting from whole blood by density centrifugation, e.g. using Ficoll®. Using such a specific biological sample or preparation as the starting material may (further) improve the reliability and/or accuracy of the method for the invention.

In a further embodiment according to the invention the amount of cytokine present as protein in (a subset of) blood cells is determined. Peripheral blood mononuclear cells (PBMC) are isolated by density gradient centrifugation. Optionally the isolated cells are cryopreserved for analysis at a later moment in time. Following routine methodology cells are cultured, fixated and cytokines are intracellularly stained using anti-cytokine antibodies followed by analysis, for instance by flowcytometry.

The treatment with IFN-β, which according to one aspect of the invention as such does not form part of the exclusive rights claimed herein, is carried out following the judgement and instructions of a medical expert along the lines of presently established dosage regimes. As an example, a patient suffering from relapsing remitting multiple sclerosis may receive on alternate days a subcutaneous injection of 8.10⁶ international units IFN-β1b. However, any route of administration, such as for instance intra-muscularly or orally, and any dosage regime involving the administration of IFN-β to MS patients fall under the scope of the invention. The therapy may also be carried out with other forms of IFN-β, such as IFN-β1a, or a combination of different forms of IFN(-β).

The following values may be considered prognostic for determining the success rate of treatment of multiple sclerosis. Patients that benefit from treatment are termed responders, whereas patients that do not benefit from treatment are identified as non-responders. The geometric means of mRNA encoding IL-12p35, IL-18, TGF-β and/of IL-10 can be determined in blood samples from a representative group of healthy persons, and these mean values can be used as the abovementioned reference value, i.e. compared with the corresponding values in blood samples from (suspected) MS patients.

For instance, if IL-12p35 mRNA (e.g. in step b) of a method above) is at least 30% higher than the geometric mean in the healthy group, this usually correctly identifies 80% of the non-responders. If IL-12p35 is smaller or equal to the geometric mean, 81% of the responders will usually be correctly identified.

For IL-18, a measured value that is at least 30% higher than the geometric mean in the healthy group will usually identify 50% of the non-responders correctly; whereas a value that is at least 20% lower than the geometric mean in the healthy group identifies 75% of the responders correctly.

For TGF-β, a measured value that is at least 120% higher than the geometric mean in the healthy group will usually identify 60% of the non-responders correctly; whereas a value that is at least 30% lower than the geometric mean in the healthy group will usually identify 81% of the responders correctly.

For IL-10, if the value measured on day 30 of treatment (e.g. in step d of a method above and/or in step e) of the combined method mentioned above) represents at least a 2-fold increase compared to the value measured prior to treatment (e.g. step b of the second method and/or the combined method above), this will usually identify almost 94% of the non-responders correctly. If said increase is less than a factor 2, this will usually correctly identify 70% of the responders.

Furthermore, statistical analysis of combined values may increase the reliability of determining the success rate of treatment even further. For instance, in patients that can be expected to benefit from treatment, the ratio IL-12p35 mRNA to IL-10 mRNA in step b) of the combined method will usually be smaller than 10, typically smaller than 6.5; whereas in patients that can be expected not to benefit from treatment, this ratio will usually be greater than 6.5, typically greater than 10. After one of the above indicated periods of time, preferably after approximately 1 month, of treatment with IFN-β this ratio, i.e. as determined in step e) of the combined method described above, may be somewhat increased in patients that will benefit from treatment, but will usually still be smaller than 10, whereas in patients that can be expected not to benefit from treatment, this ratio will be strongly decreased and is smaller than 10 and may be even smaller than 5.

Also, (the reliability and/or the predictive value of) the method of the invention may be further improved by using specific biological samples or preparations as the starting material for the method for the invention. For instance, as mentioned above, instead of one or more samples of whole blood, a specific part, fraction or preparation of whole blood may be used, such as a fraction containing a specific cell population; for instance a blood fraction or blood preparation containing (a population of) peripheral blood mononuclear cells such as leukocytes.

It will be clear to the skilled person that the above data is such that it allows predictions to be made in individual cases. As such, the value(s) obtained for an individual patient according to the invention may be of some help to the clinician in determining the manner and course of (further) treatment. Also, the method of the invention, and/or one or more of the values obtained therewith, may be of use in assessing and/or comparing the effectiveness of other methods, regimens, active substances, pharmaceutical compositions, etc. for the treatment of (different forms of) MS - e.g. different from the treatment with IFN-β referred to hereinabove - including such treatments as are available at the date of filing and/or as may become available in future.

For instance, the method of the invention might be useful in predicting and/or assessing the effectiveness of treatment(s) of MS which include, but are not limited to:
- treatment with (i.e. administration of) other interferons including type 1 interferons (e.g. IFN-beta, IFN-alpha);
- treatment with interleukins (e.g. interleukin-4, interleukin-10);
- treatment with cytokines (e.g. transforming-growth-factor-beta), chemokines or available antagonists thereof (including cytokine-directed antibodies);
- treatment with cytokine and chemokine receptor antagonists or agonists;
- treatment with (other) biological response modifiers;
- treatment with inhibitors of inflammation, including but not limited to asperin and pentoxyfylline;
- treatment with vitamins;
- treatment with hormones, such as peptide and steroid hormones, including but not limited to neuropeptides and corticosteroids;
- treatment with glatimeracetate;
- treatment with (other) compounds that can interact with cells involved in the immune system (such as antigen-presenting cells including but not limited to dendritic cells, macrophages and monocytes, T cells, B-cells, NK cells, etc.); for instance suitable antibodies and antagonists, including but not limited to antigens and antagonists blocking co-stimulatory or assessory molecules on the aforementioned cells/cell types - e.g. anti-CD40 ligand, anti-alpha4-integrin or other cell surface molecules involved in (the causation of) MS - as well as T cell receptor agonists or antagonists such as alternate peptide ligands; and/or
- treatment with autoantigens (e.g. myelin-associated proteins); or any suitable combination thereof.

In essence, when used to determine the effectiveness of such alternative treatments of MS, the method of the invention will be carried out as described hereinabove, i.e. by determining the amount of (mRNA encoding, or protein of) one or more of the abovementioned cytokines before treatment is initiated and/or after a certain period of treatment; e.g. according to the aspects as described hereinabove. It will be clear that, if the invention is used to assess and/or predict the effectiveness of such an alternative treatment, when hereinabove or in the claims reference is made to *"treatment with interferon-β* ", this should be read as treatment with the appropriate active principle used according to the alternative of treatment.

Also, the method of the invention could possibly be used to determine and/or predict the success rate of treatment(s) of different forms of multiple sclerosis, including but not limited to relapsing-remitting MS, secondary progressive MS, progressive relapsing MS, primary progressive MS; which treatment may be with interferon(s), in particular interferon-beta (IFN-β) species such as IFN-β1b and IFN-β1a, as mentioned hereinabove; may be one of the other treatments listed above; or another suitable treatment that may be available at the date of filing and/or that may become available in future.

The figures and following Experimental Parts will describe the invention in more detail without limiting its scope in any way.

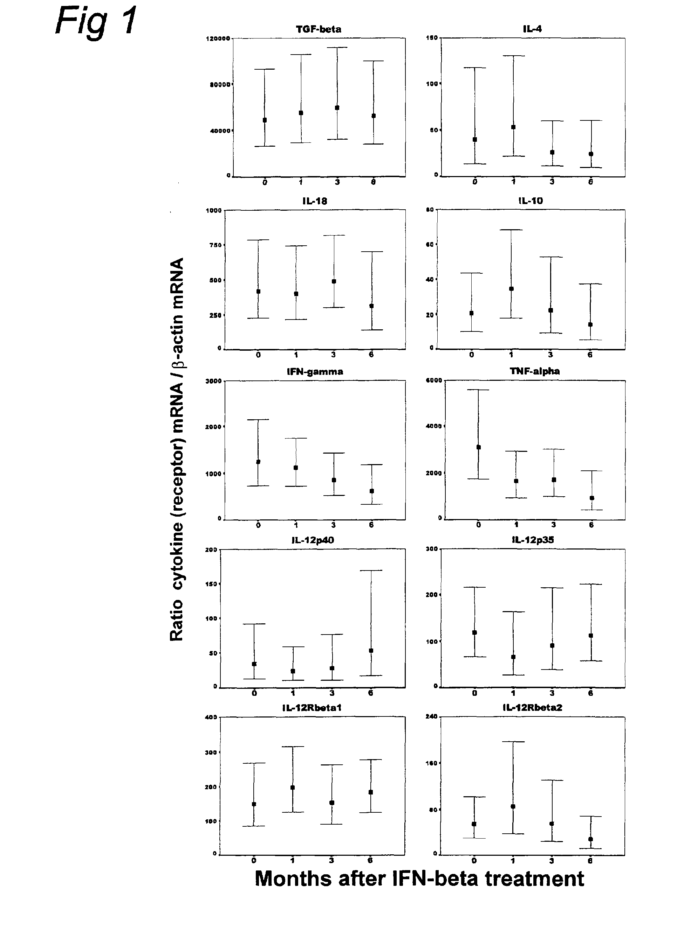

Fig. 1 are graphs of the ratio cytokine mRNA over β-actin mRNA vs months after IFN-β treatment showing that IFN-β treatment transiently increases IL-10 and IL-12Rβ2 chain mRNA and decreases TNF-α mRNA in relapsing-remitting MS. Twenty-six RR MS patients were treated with IFN-β1b and mRNA expression in whole blood cells was quantified at baseline and after 1, 3 and 6 months of treatment. The geometric means with its 95% confidence intervals are shown. The mRNA data are expressed as ratio of cytokine(receptor) mRNA over β-actin mRNA, all determined in fg standard equivalents (or arbitrary units for IL-18, IL-12Rβ1 and β2). Statistical analysis as described under the material and methods (infra), showed changes in TNF-α (p=0.001), IL-10 (p=0.057) and IL-12Rβ2 (p=0.054) mRNA for the total group.

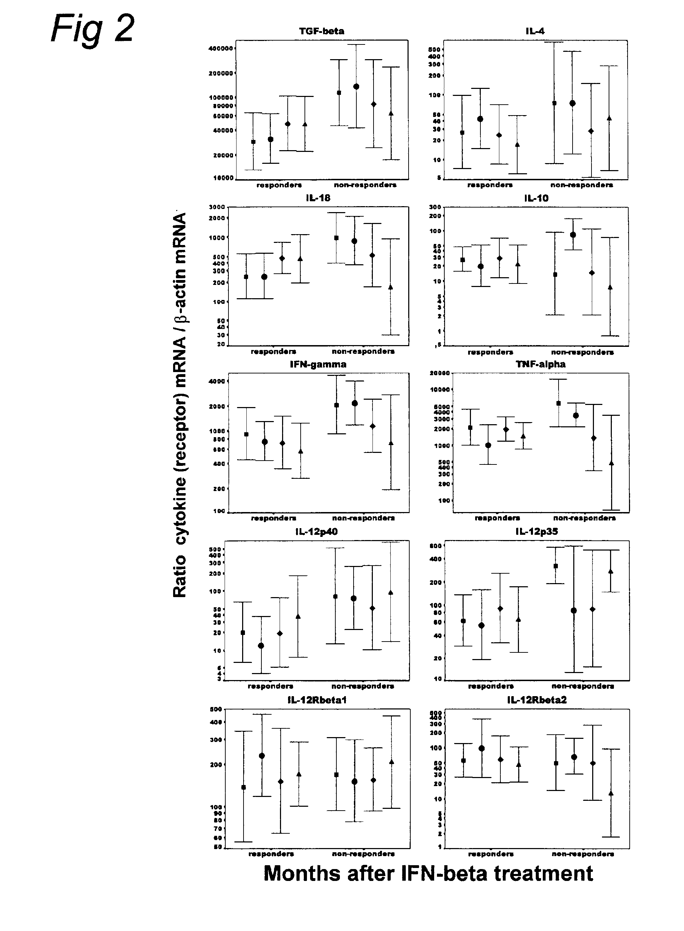

Fig. 2 are graphs of the ratio cytokine mRNA over β-actin mRNA vs months after IFN-β treatment showing that IFN-β treatment increases IL-10 and decreases TNF-α and IL-18 mRNA only in the clinical non-responders. Based on EDSS-progression, number of relapses and number of steroid interventions in the 2 years before and the 2 years after start of treatment, patients were subjectively classified as responders (16 patients) or non-responders (10 patients) to IFN-β1b. The geometric means with its 95% confidence intervals are shown. Different symbols were used to indicate the different months of treatment; the square represents geometric means on the baseline, the circle, diamond and triangle represent the geometric means on 1, 3 and 6 months after start of treatment respectively. The mRNA data are expressed as ratio of cytokine (receptor) mRNA over β-actin mRNA, all determined in fg standard equivalents (or arbitrary units for IL-18, IL-12Rβ1 and β2). Statistical analysis showed that none of the measured mRNAs changed in the clinical responders in response to treatment. Treatment related changes were found in the clinical non-responders for IL-10 (p=0.014), IL-18 (p=0.015) and TNF-α (p=0.004) mRNA.

Fig. 3. shows that cytokine (receptor) mRNAs are differently expressed in unstimulated whole blood cells of controls, relapsing remitting, secondary and primary progressive MS patients. Sixteen persons were included in each group; the geometric means with their 95% confidence interval are shown for each immunological parameter measured in each group. Statistical analysis showed significant differences between the 4 groups in the anti-inflammatory cytokines IL-10 (p=0.001), TGF-β (p≤0.0001) and IL-4 (p=0.023) and in the pro-inflammatory cytokines IFN-γ (p≤0.0001), IL-12p40 (p≤0.0001) and IL-18 (p≤0.0001). Moreover, this analysis showed differences with respect to IL-12Rβ1 and IL-12Rβ2 (both p≤0.0001).

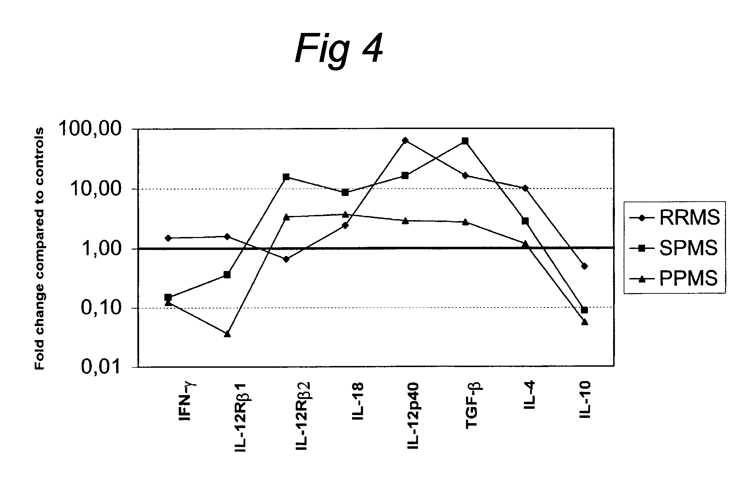

Fig. 4 displays that PP MS patients do not show increased levels of pro-inflammatory cytokines. Immunological data from the MS subgroups as shown in Figure 3 were normalized by calculating the fold difference from the geometric mean of the healthy controls. All cytokine (receptor) mRNA expression levels, which were on average 10 times higher or lower than the levels of the controls, were considered of importance.

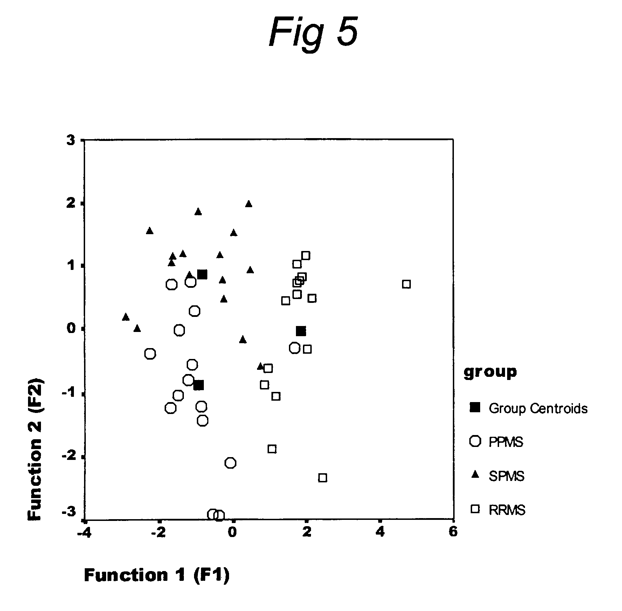

Fig. 5. shows that IFN-γ, IL-12Rβ2 and TGF-β mRNA discriminate between RR, SP and PP MS patients. Discriminant analysis was performed including all mRNA data. IFN-γ and IL-12Rβ2 mRNA discriminate RR patients from SP and PP patients (function 1: F1=-2.156 + 0.559^{*}lnIFN-γ- 0.538^{*}lnIL-12Rβ2, Table 7). TGF-β mRNA discriminates SP from PP patients (function 2: F2= -6.317 + 0.501*InTGF-β, Table 7).

### EXPERIMENTAL PART I

IFN-β treatment has been shown to be effective in relapsing-remitting multiple sclerosis (RR MS). Although its mechanism of action is still unclear, a downregulatory effect on pro-inflammatory cytokines and a stimulatory effect on anti-inflammatory cytokines have been proposed. Different response patterns to IFN-β are discriminated between clinical responders and non-responders, based on EDSS-progression, number of relapses and number of steroid interventions in the 2 years prior to and following initiation of treatment. This was done by studying whether the expression of mRNAs encoding IL-12, IL-12 receptor chains, IL-18, TNF-α, IFN-γ, IL-10, IL-4 or TGF-β in unstimulated whole blood of 26 RR MS patients changed during 6 months of IFN-β1b treatment. In the total group, only a transient increase in IL-12Rβ2 and IL-10 mRNA was observed after 1 month of treatment, whereas TNF-α mRNA was decreased after 3 and 6 months. Only the IL-10, TNF-α and IL-18 response patters to IFN-β1b treatment differed between responders and non-responders. Surprisingly, clinical responders displayed no change in these cytokines, whereas the transient increase in IL-10 mRNA and the decrease in TNF-α mRNA occurred in the clinical non-responders; the same was true for a decrease in IL-18 mRNA. Higher baseline levels for IL-12p35 mRNA were found in the non-responders. This marker predicts the clinical outcome in ±81% of the patients correctly (p=0.0002).

Following the demonstration of IFN-β being able to suppress disease activity in relapsing-remitting multiple sclerosis (RR MS), increasing numbers of patients are treated with this immunomodulating drug. However, the precise mechanism of action of IFN-β is still largely unknown. Several activities have been claimed including inhibition of antigen presentation, inhibition of diapedesis of leukocytes through the blood-brain-barrier, downregulation of pro-inflammatory cytokines and activation of anti-inflammatory cytokines. Several clinical studies have shown that pro-inflammatory cytokines like TNF-α are downregulated either at the protein or mRNA level, but there are contradictory results for IFN-γ. Furthermore, an increase has been demonstrated for IL-4 and especially IL-10 at both expression levels. Hence one of the major effects of IFN-β might be the control of inflammation.

MS is widely regarded to comprise an inflammatory process in which macrophages are stimulated to become involved in demyelination after their activation by Th1 cells specific for myelin-antigens. However, also Th2 cells secreting IL-4 and other effector T cells capable of secreting anti-inflammatory cytokines such as TGF-β and IL-10 are supposed to play an important role in control of the disease. Therefore, factors that determine whether a naive T cell differentiates into a pro-inflammatory Th1 cell, an anti-inflammatory Th2 or Th3 cell or a putative regulatory Tr1 cell are likely to be of importance in MS. This has already been demonstrated for IL-12 (a Th1 inducer) and IL-10 (a Th1 inhibitor), in as much as the former is upregulated in active MS and prior to relapse whereas the latter is associated with the remitting phase. Little is known with regard to effects of IFN-β on IL-12 and IL-18 - both cytokines contributing to Th1 polarization - in the context of MS. Interestingly, IFN-β has been demonstrated to be a crucial factor in upregulating the IL-12Rβ2-chain *in vitro,* which is required for the formation of a functional receptor in conjunction with a β1-chain. On the basis of these findings it would be expected that IFN-β would promote Th1 activity which is at odds with the current view that the latter contributes to disease progression. Hereinbelow, whole blood was studied in terms of mRNA expression encoding a variety of markers including novel parameters, i.e. IL-12p40, IL-12p35, IL-18, IL-12Rβ1 and IL-12Rβ2. This was done by studying material from 26 RR MS patients, derived from baseline and 1, 3 and 6 months after start of the treatment with IFN-β1b. These are the first clinical results based on EDSS-progression, number of relapses and number of steroid interventions in the 2 years prior to and following initiation of treatment showing certain parameters are predictive for the efficacy of IFN-β1b.

### Material and Methods

### Patients

Thirty consecutive MS patients who started treatment with recombinant interferon-β1b (Betaferon, Schering AG, Berlin, Germany) at the Department of Neurology, Free University Hospital, Amsterdam were included in this study. Inclusion criteria were: definite MS according to Poser criteria, relapsing-remitting disease type, at least 2 exacerbations in the 2 years before the start of treatment and mild to moderate disability (Expanded Disability Status Scale (EDSS) score of ≤ 6.0). Treatment consisted of subcutaneous injection of 8 million international units of IFN-β1b on alternate days. EDTA blood was sampled at fixed time-points: 0 (start of treatment, baseline), 1, 3 and 6 months after the start of treatment. Other relevant data such as relapses, steroid interventions and EDSS scores, at baseline and after 1, 3, 6 and 24 months after start of treatment, were also collected. Analyses were carried out on 26 patients (22 female, 4 male). The mean age of these patients was 37.5 ± 7.4 years and the mean disease duration was 7.0 ± 5.2 years. Four of the 30 patients were not included in the analysis. Three of them discontinued treatment early; neither complete clinical data nor a sufficient number of blood samples was obtained. One patient was excluded because breast cancer was diagnosed after about one month on IFN-β1b treatment.

Based on EDSS-progression, number of relapses and number of steroid interventions in the 2 years prior to and following initiation of treatment, patients were classified as responders or non-responders to IFN-β1b. Of the 26 patients, 16 were responders and 10 non-responders.

The demographic and treatment outcome characteristics for these patients are given in Table 1. This table demonstrates that the non-responders were comparable to the responders in the 2 years before treatment. Furthermore it shows that -as could be anticipated- the non-responders had a significantly higher number of relapses and high dose intravenous methylprednisolone (IVMP) interventions as well as more progression on the EDSS-scale than the responders during the 2-year treatment period with IFN-β1b. In the 3 months prior to start of treatment, 8 patients received IVMP, of whom 5 ultimately were classified as responders. During the first 6 months of treatment (the blood sampling period) 6 patients received IVMP, 4 of whom were non-responders.

**Table 1:**

| Demographic and treatment outcome characteristics of RR MS patients | | |
|---|---|---|
| **Clinical responsiveness** | **responders** | **non-responders** |
| number of patients | 16 | 10 |
| mean age (years ± SD) | 36.1 ± 7.8 | 39.9 ± 6.4 |
| disease duration (years ± SD) | 6.3 ± 5.1 | 8.1 ± 5.6 |
| mean number of clinical relapses in 2 years before start of treatment | 3.4 ± 1.0 | 3.4 ± 1.3 |
| mean number of clinical relapses in 2 years after start of treatment | 0.7 ± 0.8 | 2.6 ± 1.1^{a} |
| mean number of MP treatments in 2 years after start of treatment | 0.4 ± 0.8 | 2.1 ± 1.3^{b} |
| EDSS at baseline | 3.9 ± 1.4 | 4.1 ± 1.6 |
| EDSS progression in 2 years after start of treatment | 0.2 ± 0.7 | 1.3 ± 0.9^{c} |

| | | |
|---|---|---|
| ^{a} p#0.0001, ^{b} p=0.001, ^{c} p=0.002 (all for differences between responders and non-responders); MP: methylprednisolone | | |

### mRNA isolation and cDNA synthesis

EDTA blood was collected by venapuncture and kept at 4°C for maximal 18 hours. First, the erythrocytes were lysed after incubation with a buffer containing 155 mM NH₄Cl, 10 mM KHCO₃ and 0.1 mM EDTA (PH 7-7.5) for 10 minutes at 4°C. mRNA was subsequently isolated from the remaining cells using RNAzolB (Campro Scientific, Veenendaal, The Netherlands) according to the manufacturer's instructions and the samples were stored at 80°C. Three µg of RNA was reverse transcribed into cDNA using the Reverse Transcription system kit (Promega, Madinson, WI) and the products were subsequently stored at -20°C.

### Non-competitive RT-PCR

cDNA derived from whole blood mRNA of the patients was quantified in a semiquantitative fashion. To this end the cDNA was amplified in triplicate. Quantification was enabled employing simultaneous amplification of stepfold diluted external standards. Unless indicated otherwise the multi-specific vectors pQA-1 and pQB-3 (Bouaboula et al., J. Biol. Chem. 1992;267: 21830-21838) were used. For IL-12p40 and IL-12p35, a pQA-1/IL-12 construct prepared as described previously (Visser et al., 1998) was used. A standard for TGF-β was kindly provided by H. Baelde and Dr. E. de Heer (Dept. of Pathology, Leiden University Medical Center, The Netherlands). To enable quantification of IL-18, IL-12Rβ1 and β2, cDNA was prepared after stimulation of PBMC (from a healthy donor) with 4 hours of 250 ng/ml LPS (Escherichia coli-derived, serotype 0127:B8, Sigma, St Louis, MO) for IL-18 or 3 days with 1 µg/ml PHA (HA 16, Phaseolus-species; Murex Biotech LTD, Dartford, Kent, England) for the IL-12Rbeta chains. Table 2 shows the primer sequences of the primers used for IL-4, TGF-β, IL-18, IL-12Rβ1 and β2.

**Table 2:**

| Primer sequences used | |
|---|---|
| TGF-β sense | AGCGACTCGCCAGAGTGGTTATCTT |
| TGF-β antisense | TTATGCTGGTTGTACAGGGCCAGGA |
| IL-4 sense | TGCCTCCAAGAACACAACTG |
| IL-4 antisense | AACGTACTCTGGTTGGCTTC |
| IL-18 sense | GCTTGAATCTAAATTATCAGTC |
| IL-18 antisense | GAAGATTCAAATTGCATCTTAT |
| IL-12Rβ1 sense | CCTGAAAACCCCCCACAG |
| IL-12Rβ1 antisense | GCTGAGGCATTGCCCCCA |
| IL-12Rβ2 sense | CTGCAAACTGGCCTGTATCAA |
| IL-12Rβ2 antisense | GTGCTCTCAATGATTCACTCC |

Primer sequences used for amplification of the other cDNA products have been published previously (Visser et al., 1998). The 50 µl reaction mixture that was added per tube contained PCR buffer I (50 mM KCl, 10 mM Tris/HCl pH 8.3, 2 mM MgCl₂ and 60 ng/µl BSA) when PQA-1, PQB-3, TGF-β-, IL-18-, IL-12Rβ chain-primers were used, and PCR buffer II (50 mM KCl, 10 mM Tris/HCl pH 8.3, 100 ng/µl gelatin and 2.5 mM MgCl₂; Perkin Elmer) when PQA-1/IL-12 was used, together with 0.25 mM dNTPs (Gibco BRL, Gaithersburg, MD), 12.5 pmol sense and antisense primers (Isogen Bioscience, Maarssen, The Netherlands) and 1 unit Taq DNA polymerase (Gibco BRL). After initial denaturation of 2 min at 96°C, cycling conditions were 0.5 min of denaturation at 96°C, 1 min annealing at 55°C (60°C for IL-12Rβ1 and β2 amplification) and 1 min elongation at 72°C, repeated during 30 cycles for β-actin and 35 cycles for the cytokines and receptors and a final elongation step of 4 min at 72°C (Perkin Elmer 9600 PCR apparatus). For IL-4 and TGF-β cycling conditions after the initial 2 min of denaturation at 96°C were: 0.5 min of denaturation at 96°C, 1 min annealing at variable temperature and 1 min elongation at 72°C. For the first 2 cycles the annealing temperature was 58°C (65°C for TGF-β, and subsequently 1°C lower after every 2 cycles until 54°C (61°C for TGF-β followed by 10 cycles at 53°C (60°C for TGF-β) and the last 20 cycles at 52°C (59°C for TGF-β. The PCR products were separated on a 2% agarose gel and stained with ethidium bromide for β-actin, TNF-α, IL-18 and TGF-β amplified products. Visualization of the other PCR products required enhancement employing SYBR Green I nucleic acid gel stain (Molecular Probes Europe BV, Leiden, The Netherlands). The amount of PCR product was determined by densitometric imaging using the Bio-1D digital imaging system version 6 (Vilber Lourmat, Marne la Vallée, France). To correct for differences in initial cell numbers, as well as the efficiency of mRNA isolation and cDNA synthesis, the amounts of cytokine cDNA are expressed in fg standard equivalents (or arbitrary units for IL-18, IL-12Rβ1 and β2) per fg β-actin standard equivalents. All shown data were normalized in this manner and multiplied with 10⁶.

### Isolation of peripheral blood mononuclear cells (PBMC)

Within 24 hours after puncture, PBMC were isolated by Histopaque 1077 (Sigma diagnostics, St. Louis, MO) density gradient centrifugation, cryopreserved in Iscove's Modified Dulbecco's Medium (IMDM, Gibco BRL, Gaithersburg, MD) supplemented with 20% FCS and 10% DMSO (Merck, Darmstadt, Germany) and stored at -196°C until use.

### Intracellular staining of cytokines

After quick thawing of the cells at 37°C, PBMC were washed and cultured for 4 or 22 hours at a final density of 7 to 9x10⁵ cells/well in 6 well-flat bottom plates (Corning Costar Corporation, Cambridge, MA, USA) in IMDM, supplemented with 10% fetal calf serum (FCS, Sebak, Aidenbach, Germany), 5x10⁻⁵M 2-mercaptoetanol, 100 U/ml penicilline and 100 µg/ml streptomycine in the presence of 10 µg/ml Brefeldin A (Sigma, St.Louis, MO, USA). To enable the spontaneous accumulation of IL-10 in monocytes or T cells, PBMC were incubated in medium only in the presence of Brefeldin A during 4hrs or 22 hrs respectively. Longer incubation of PBMC led to unacceptable loss of monocytes. After incubation cells were washed 3 times and incubated with mouse-a-human-CD4-rpe-cy5, mouse-α-human-CD8-rpe-cy5 or mouse-a-human-CD14-rpe-cy5 antibodies (Coulter Immunotech, Marseille Cedex, France). Next, cells were washed and fixated with 2% formaldehyde (UCB, Leuven, Belgium) in PBS for 20 minutes at 4°C. Subsequently, cells were washed 3 times, and intracellular cytokines were stained by incubation (30 minutes at 4°C) with 2.5 µg/ml mouse-α-human-IL-4-PE, 1.25 µg/ml mouse-α-human-IFN-γ-FITC (both from Becton Dickinson, San Jose, CA) or 10 µg/ml rat-α-human-IL-10-PE (Caltag Laboratories, Burlingame, CA) each diluted in PBS/1% FCS/0.1% saponin (ICN Biomedicals, Aurora, Ohio). To control for non-specific staining, the following isotype controls were included: 2.5 µg/ml mouse IgGl-Pe (Dako, Denmark), 1.25 µg/ml mouse IgG2b-FITC (Dako) and 10 µg/ml rat IgGl-PE (Caltag). After incubation, cells were washed 3 times with PBS/1% FCS/0.1% saponin and analysed on the FACScalibur.

The possibility to stain IL-10 intracellularly is not restricted to the method described above, but can also be achieved using commercial available kits. For instance, the "Fix and Perm method" (Caltag, Laboratories, Burlingame, CA) is suitable for intracellular staining of cytokines when performed according to indications of the manufacturer.

### Statistical Methods

Statistical analysis was performed by using the SPSS 8.0 package (SPSS Inc, Chicago, IL) for MS-Windows. To test possible differences in clinical data between responders and non-responders, the Independent Samples T-test was used. In order to establish a normal distribution of the data, a logarithmic transformation of the cytokine (receptor) mRNA data was performed and further used during all statistical analyses. The General Linear Model (GLM) analysis of variance module (Repeated Measures) was used to test the total group of patients for changes in cytokine mRNA levels between the different months. If this analysis showed a difference (p<0.1), post-hoc analysis was performed using the Paired-Samples T-test in order to detect where the changes took place. Furthermore, the Multivariate Analysis under GLM was used to compare cytokine levels on all 4 data points between responders and non-responders. If this overall test showed any differences (p<0.05), the Independent Samples T-test was performed in order to detect on which time points the cytokine levels were different. Repeated Measures analysis was further used to test the separate groups of responders or non-responders for changes in cytokine mRNA levels between the different months. If this analysis showed a difference (p<0.05), post-hoc analysis was performed using the Paired-Samples T-test in order to detect where the changes took place. In addition, the Repeated Measures analysis was used to test whether changes in cytokine mRNA profiles differed between responders and non-responders. For Repeated Measures analysis the within-subject variable has 4 levels (baseline, 1, 3 and 6 months after start of treatment) and respondency was used as between-subject factor. To get further insight in the differences between responders and non-responders, Logistic Regression was performed to test at which time point cytokine expression was predictive for responsiveness. Statistical outcomes with a p<0.05 or p<0.1 were considered to be significant or to indicate a trend, respectively.

### Results

### Effect of IFN-β1b treatment on cytokine and IL-12R mRNA expression in whole blood cells of RRMS patients

Expression levels of several pro- and anti-inflammatory cytokines and the IL-12R chains (geometric means and 95% confidence intervals) at baseline and after 1, 3 and 6 months of treatment with IFN-β1b mRNA are given in Fig. 1. In order to detect changes in cytokine (receptor) expression, all time points were included for the first statistical analysis. As expected, IFN-β1b had a downregulatory effect on TNF-α mRNA (p=0.001). Post-hoc analysis demonstrated a trend 1 and 3 months (p=0.072 and p=0.056 respectively) after start of treatment and a significant decrease after 6 months (p=0.010; all compared to baseline). IFN-β1b treatment did not appear to have an effect on the other pro-inflammatory cytokines. Although IFN-β1b had no effect on the IL-12Rβ1 chain, changes in IL-12Rβ2 chain expression showed a trend (p=0.054). More detailed analysis showed a transient increase after 1 month, followed by a decrease until 6 months of treatment (p=0.025; between month 1 and month 6). As far as the anti-inflammatory cytokines were concerned, IFN-β1b had no demonstrable effect on TGF-β and IL-4 mRNA during 6 months of treatment. In contrast, a trend towards a change in IL-10 mRNA was observed (p=0.057). Further analysis demonstrated a transient increase 1 month after the start of treatment (p=0.043; between baseline and month 1).

### Differences between responders and non-responders

Based on a comparison of EDSS-progression, number of relapses and number of steroid interventions before and during treatment with IFN-β1b, patients were classified as responder (16 out of 26; 61.5 %) or non-responder (10 out of 26; 38.5 %). Fig. 2 shows both for responders and non-responders the geometric means with 95% confidence interval of cytokine (receptor) mRNAs at baseline and after 1, 3 and 6 months of treatment. A multivariate comparison of the mRNA expression levels between these groups revealed that non-responders differed significantly from responders with respect to IL-12p35 (p=0.010) and TNF-α (p=0.014) mRNA expressed during the follow-up period of 6 months (Table 3). Furthermore, a trend towards differences in mRNA levels of IFN-γ (p=0.097), IL-18 (p=0.055), IL-10 (p=0.068) and TGF-β (p=0.063) was observed (Table 3). The Independent Samples T-test was performed to establish on which months the differences in IL-12p35 and TNF-α mRNA were most apparent between responders and non-responders (Table 3). At baseline, the non-responders showed a trend towards higher TNF-α mRNA (p=0.084) and these levels were still higher after 1 month of treatment (p=0.026). After 3 and 6 months of treatment, these TNF-α mRNA levels appeared equal to those of the responders, suggesting that levels declined in the clinical non-responders in response to treatment. Higher levels for IL-12p35 mRNA were observed in the non-responders at the baseline (p=0.003) and after 6 months of treatment (p=0.028). On the intervening months the IL-12p35 mRNA levels in the non-responders were not different from those in the responders, suggesting more complex response patterns.

**Table 3:**

| Differences between responders and non-responders during the 6 months follow-up | | | | | |
|---|---|---|---|---|---|
| **Cytokine** | **Multivariate analysis** | **baseline** | **1 month** | **3 months** | **6 months** |
| IL-12Rβ1 | p=0.656 | - | - | - | - |
| IL-12Rβ2 | p=0.689 | - | - | - | - |
| IL-4 | p=0.771 | - | - | - | - |
| IL-12p40 | p=0.322 | - | - | - | - |
| IL-12p35 | p=0.010 | p=0.003 | n.s. | n.s. | p=0.028 |
| IFN-y | p=0.097 | - | - | - | - |
| IL-18 | p=0.055 | - | - | - | - |
| IL-10 | p=0.068 | - | - | - | - |
| TGF-β | p=0.063 | - | - | - | - |
| TNF-α | p=0.014. | p=0.084 | p=0.026 | n.s. | n.s. |
| n.s.: not significantly different -: not tested | | | | | |

We next investigated whether cytokine expression in the non-responders was differently affected by IFN-β1b treatment as compared to the responders. The following cytokines showed significantly different changes between responders and non-responders during the first 6 months after initiation of treatment: IL-10 (p=0.017), IL-18 (p=0.009) and TNF-α (p=0.002). Fig. 2 shows that IL-18 mRNA did not significantly change in the responders, whereas this cytokine decreased in the clinical non-responders after IFN-β1b treatment (non-responders: month 0-6, p=0.079; month 1-3, p=0.033, month 3-6, p=0.038). Unexpectedly, TNF-α mRNA showed no change in the responders, whereas a strong decrease was found in the non-responders (month 0-3, p=0.037, month 0-6, p=0.015, month 1-6, p=0.036). Surprisingly, IFN-β1b did not have an effect on IL-10 mRNA in the responders, as opposed to a transient increase in the non-responders that was followed by a decrease (increase month 0-1, p=0.037; decrease month 1-3 and 1-6, p=0.051 and p=0.029).

In conclusion, the non-responders were accountable for the decrease in TNF-α mRNA as well as the transient increase in IL-10 mRNA as observed in the total group. In contrast, both responders and non-responders showed a transient increase in the IL-12Rβ2 chain after 1 month of treatment. Furthermore, non-responders and responders showed opposite reaction patterns for IL-18 mRNA after treatment, i.e. no change in the responders and a decrease in the non-responders.

### Baseline IL-12p35 mRNA predicts responsiveness to IFN-β1b treatment

We next analyzed which parameters at baseline would have predictive value for clinical outcome, including mRNA data of all 10 tested parameters on the baseline. When all these 10 parameters were included, the overall predictive value showed a trend (p=0.080). After testing each of the 10 parameters separately (univariate analysis), significant outcomes for TGF-β (p=0.026), IL-18 (p=0.025) and IL-12p35 mRNA (p=0.0046) were demonstrated as well as a trend for TNF-α mRNA (p=0.078). Further analysis (forward stepwise regression) showed that the mRNA levels of IL-12p35 on the baseline were the best predictor for responsiveness (p=0.0002), i.e. high IL-12p35 mRNA levels are correlated with non-responsiveness. With this marker 13 out of 16 responders (81.3%) and 8 out of 10 non-responders (80.0%) were correctly predicted, responsible for a correct prediction of on average 80.8%.

### Effect of IFN-β treatment on cytokine production in peripheral blood mononuclear cells (PBMC)

Further studies into effects of IFN-β on different subsets (CD4⁺ and CD8⁺ T cells, monocytes) showed that before the start of treatment both clinical responders (p=0.034) and nonresponders (p=0.016) had decreased numbers of CD4⁺ T cells that spontaneously accumulated IL-10 as compared with healthy controls. This difference could not be attributed to lower numbers of CD4⁺ T cells within PBMC of responders (n=7) and nonresponders (n=7) compared to controls (n=6). Remarkably, only the clinical responders showed a change in such cells (p=0.043). Further post-hoc analysis revealed that the responders showed a trend to an increase after 3 months (month 0-3; p=0.099), and after 6 months of treatment a significant increase to normal values could be observed (month 0-6; p=0.002). In contrast, the numbers of IL-10 producing CD4⁺ T cells remained low in the clinical responders. After 2 years of treatment, the clinical responders demonstrated a 90% reduction of exacerbations compared to the 2 years before the initiation of treatment. This suggests that the CD4⁺ T cell subset that spontaneously produces IL-10 plays an important role in regulation of disease. Because IL-10 is known for its downregulatory capacities, it is likely that the therapeutical effect of IFN-β can be explained by stimulation of IL-10 producing T cells in general, and stimulation of the IL-10 producing CD4⁺ T cells subset in particular.

It appeared that IFN-β has contrasting effects on different cell subsets. The numbers of IL-10 producing monocytes that were increased in both responders (p=0.033) and nonresponders (p=0.006) on the baseline as compared to controls, decreased in the total group of RR patients during treatment (p=0.050). Post-hoc analysis demonstrated that the numbers became equal to levels found in the healthy controls (month 0-6, p=0.050; month 3-6, p=0.012). Therefore, the enhancement of the number of IL-10 positive CD4⁺ T cells in the clinical responders that occurred simultaneously with a decrease in the fraction of IL-10 producing monocytes may explain why no change could be observed on the IL-10 mRNA level in the clinical responders after 6 months of treatment.

The transient increase in IL-10 mRNA in the clinical nonresponders after 1 month of treatment seems to be not related to an increase in the number of IL-10 producing T cells or monocytes. It is likely that this transient increase occurs in an as yet unidentified cell subset in whole blood.

### Discussion

Although it has been shown that IFN-β greatly influences disease activity in RR MS, its mechanism of action is largely unknown. It is however generally assumed that the cytokine network represents an important target. The use of unstimulated PBMC may be most appropriate for a proper appreciation of the relationship between cytokines and clinical activity. Here we analyzed mRNA expression in unstimulated whole blood cells of 26 RR MS patients treated with IFN-β1b. IFN-β did not affect either chain of the IL-12 molecule, confirming previous observations obtained by mitogenic stimulation. Further detailed analyses revealed no change in responders or non-responders. As predicted by *in vitro* data concerning the effects of IFN-β, the IL-12Rβ2 chain was transiently upregulated in both subgroups. Therefore IFN-β may form a bias for enhanced Th1 responses. Since neither IFN-γ nor TNF-α mRNA levels were found to be increased, this does not seem to be the case. On the contrary, treatment rather decreased TNF-α mRNA in the patients. Unexpectedly, we found that only the non-responders were responsible for the decrease in TNF-α mRNA, whereas no change was observed in the responders. Thus, despite the expected beneficial downregulating effect of IFN-β therapy on pro-inflammatory cytokines, no decreases were observed in IL-12, IL-18, TNF-α and IFN-γ mRNA in the responders. In contrast, we could only demonstrate a decrease for IL-18 and TNF-α mRNA in the non-responders. In this respect it is of interest that treatment with anti-TNF-α monoclonal antibodies in 2 rapidly progressive MS patients resulted in increased MRI activity. Interestingly, preliminary results with intracellular FACS-staining of spontaneous TNF-α production in monocytes also show that this cytokine increases in the responders, whereas it decreases in the non-responders. These data suggest that the assumption that a decrease in TNF-α is beneficial in MS may require revision and furthermore, that TNF-α may have stimulatory properties in a regulatory circuit.

Upregulation of anti-inflammatory cytokines is another explanation for the mechanism of IFN-β therapy. In this respect IL-10 has been shown to be preferentially enhanced, whereas no effect is observed on TGF-β mRNA. Likewise, we showed in this study a transient increase in IL-10, but no change in TGF-β or IL-4 mRNA. This increase in IL-10 mRNA after 1 month of therapy was followed by a decrease after 3 and 6 months of treatment. These changes in IL-10 confirm fluctuations found in corresponding other plasma samples. Unexpectedly, we found that the transient increase in IL-10 mRNA took only place in the non-responders. This transient increase in IL-10 mRNA in the non-responders may explain the subsequent decrease seen in TNF-α and IL-18 mRNA after 3 and 6 months of treatment. Since the non-responders had more clinical relapses than the responders did, we investigated whether methylprednisolone interventions during the follow-up period could explain these changes only seen in this subgroup of patients. During the 6 months of treatment, 2 and 5 short courses of intravenous methylprednisolone were given to the responders and non-responders respectively. Influence of methylprednisolone on cytokine mRNA was only considered to be present when the treatment took place within 1 month before blood sampling; only 3 methylprednisolone interventions in the non-responders fulfill this criterion (one before 1, 3 or 6 months of treatment). When these data points were excluded from the study, no different response patterns where observed for the non-responders, indicating that alternative explanations are required to clarify the different clinical responses to IFN-β therapy.

In conclusion, the patients in whom IFN-β had a beneficial effect did not show a significant decrease in pro-inflammatory cytokines or a significant increase in anti-inflammatory cytokines, as expressed in total blood cells.

The measurement of IL-12p35 and IL-10 mRNA on the baseline and after 1 month of treatment is very useful for the discrimination between clinical responders and nonresponders to IFN-β treatment. In addition to this, the measurement of the number of IL-10 producing CD4⁺ T cells is also very useful for the discrimination between responders and nonresponders. Using only the difference between month 0 and 6 with regard to the numbers of IL-10 producing CD4⁺ T cells, already a correct prediction of on average 92.3% can be achieved (p=0.003). It appeared that 7 out of 7 clinical responders (100%) and 5 out of 6 nonresponders (83.3%) were correctly predicted in this manner.

In conclusion, the correct prediction of clinical responsiveness to IFN-β therapy can be improved by adding the measurement of spontaneous IL-10 production in CD4⁺ T cells to the measurement of IL-12p35 and IL-10 mRNA (on the baseline and after 1 and 6 months of treatment).

It is of great importance to distinguish clinical responders from non-responders to IFN-β therapy already before the start of treatment. We found that IL-12p35, IL-18 and TGF-β mRNA's were increased in the non-responders at the baseline. Detailed statistical analysis showed that IL-12p35 mRNA levels had the best predictive value for clinical outcome. With these levels we were able to predict correctly 80.8% of treatment responses in our patients. We investigated whether occurrence of relapses in the 3 months before the start of treatment could explain the higher IL-12p35 mRNA levels in the non-responders. It appeared that in this period 5 out of 16 responders and 6 out of 10 non-responders had a relapse, but that the observed difference in IL-12p35 mRNA between responders and non-responders was independent of occurrence of relapse. Expression of IL-12p35 mRNA could be synonymous for functional IL-12 but since the correlation between IL-12p35 and IL-12p40 mRNA showed only a trend (p=0.086) in the non-responders on the baseline, this seems however unlikely. IL-12p35 mRNA is constitutively expressed in T and NK cells, although its function is unknown. Increased levels in the non-responders may thus be the reflection of such an increased, but yet unidentified cell subset.

### EXPERIMENTAL PART II

Recently, it was demonstrated in a longitudinal study that IL-12p40 mRNA is increased both in RR and SP patients and that IL-10 mRNA is low in SP MS in particular. Little is known about the involvement of these and other cytokines in the pathogenesis of PP MS. We evaluated in this cross-sectional study whether IL-18, IL-12p35, IL-12p40, TNF-α, IFN-γ, IL-10, IL-4, TGF-β, IL-12Rβ1 and IL-12Rβ2 mRNA expression in unstimulated whole blood showed significant differences between relapsing- remitting (RR), secondary progressive (SP) and primary progressive (PP) multiple sclerosis (MS) patients and healthy controls (n=16 in each group). All clinical subtypes showed unique mRNA expression patterns as compared to the controls. Both RR and SP patients displayed increased levels of IL-12p40, IL-18 and TGF-β mRNA compared to controls; only SP patients showed increased IL-12Rβ2 mRNA levels. Both in PP and SP patients IL-10 mRNA levels were decreased compared to RR patients and controls. However, only SP patients showed an inverse correlation between EDSS and IL-10. PP patients differed from RR and SP patients in that they had lower IL12Rβ1 mRNA without evidence for increased levels of pro-inflammatory cytokines. Our data show that the assessment of cytokine (receptor) mRNA profiles is useful to discriminate between the different clinical subtypes and suggest that different cytokines are involved in the pathogenesis of PP MS as compared to RR and SP MS.

Multiple sclerosis (MS) is widely regarded as an autoimmune disease in which macrophages are stimulated to become involved in demyelination of axons in the central nervous system after their activation by Thelperl (Th1) cells specific for myelin-antigens. Pro-inflammatory cytokines like tumor necrosis factor-alpha (TNF-α), interferon-gamma (IFN-γ) and interleukin-12 (IL-12) produced by these infiltrating cells are considered to be important contributors to disease progression in MS. Uncontrolled demyelination might be a result of lack of control by regulatory T cells (like Th2, Th3 or Tr1) secreting anti-inflammatory cytokines such as IL-4, IL-10 or transforming growth factor-beta (TGF-β). Therefore, in the context of MS it is important to study those cytokines that determine the outcome of T cell differentiation, i.e. IL-12 and IL-18 which stimulate Th1 differentiation, in addition to IL-4 and IL-10 that stimulate Th2 and Tr1 differentiation. Especially the balance between IL-10 and IL-12 may be of major importance in determining disease activity; we have recently demonstrated that in relapsing remitting (RR) MS patients IL-12p40 is upregulated prior to relapse, whereas IL-10 is low before relapse and enhancement of this cytokine is associated with the remitting phase. Moreover, this study provides evidence that the control of disease activity differs between RR patients and those in the secondary progressive (SP) phase. RR as well as SP patients show increased levels of IL-12p40 mRNA before the development of active lesions together with low levels of IL-10 mRNA. Whereas the RR patients demonstrated normal levels of IL-10 mRNA when active lesions became apparent, the SP patients showed low IL-10 levels constitutively. Little is known with regard to the involvement of cytokines in the disease process of primary progressive (PP) MS patients, who suffer from progressive disease from onset. It is likely that the etiology of disease in this patient group is different from RR and SP patients, since the PP patients show a lower female predominance, a different immunogenetic profile, considerably less (gadolinium-enhancing) lesions on brain MRI and less inflammatory lesions. These data suggest that PP patients have less inflammatory lesions and that clinical deterioration may occur by mechanisms other than progressive demyelination, for example spinal cord atrophy, axonal degeneration, and neuronal apoptosis.

The purpose of this cross-sectional study was to assess whether the different clinical subtypes of MS showed significant differences on the basis of cytokine mRNA expression in whole blood. To this end, whole blood samples of RR, SP and PP patients, 16 each, were studied in comparison with 16 healthy controls. Because we quantified the mRNA expression of several pro-inflammatory (IL-18, IL-12p35, IL-12p40, TNF-α, IFN-γ) and anti-inflammatory (IL-10, IL-4, TGF-β) cytokines in unstimulated whole blood cells, our results are likely to reflect the *in vivo* activation state of these cells as opposed to *in vitro* stimulated cells. Since the upregulation of the IL-12Rβ2-chain on naive T cells is an important step contributing to Th1 polarization, we studied this chain in conjunction with the IL-12Rβ1-chain, both being required for the formation of a functional receptor. Our data show substantial differences in cytokine (receptor) mRNA patterns between the different subgroups.

### Material and Methods

### Patients and controls

RR, SP and PP MS patients (16 in each group) of the Department of Neurology, Free University Hospital, Amsterdam were included in this study. Definite MS was ascertained according to the Poser criteria, and clinical disability was established according to the Expanded Disability Status Scale (EDSS). The demographic characteristics of these patients are given in Table 4. Two out of 16 RR MS patients had a clinical relapse 4-6 weeks before blood sampling; one of these patients was on IFN-β1b therapy since 4 months. Another RR MS patient received 10 mg of prednisone orally during blood sampling. Two out of 16 SP MS patients had a clinical relapse shortly before blood sampling; none of the PP and SP patients were under immune-modifying treatment. Sixteen healthy volunteers (9 female, 7 male) were included as a control group. The mean age of these controls was 35.4 ± 11.4 years for the females, 45.3 ± 9.1 years for the males and 39.8 ± 11.3 years for the total group.

**Table 4:**

| Demographic and clinical characteristics of the MS patients under investigation | | | |
|---|---|---|---|
| **Patient group** | **RR** | **SP** | **PP** |
| **Sex** (female; male) | 13; 3 | 8; 8 | 8; 8 |
| **mean age** (years ± SD) | 42.1 ± 5.1^{a} | 45.2 ± 7.5 | 51.3 ± 10.5^{b} |
| **mean disease duration** (years ± SD) | 8.9 ± 6.6 | 12.4 ± 7.3 | 13.1 ± 8.0 |
| **mean EDSS at intake** (± SD) | 2.3 ± 1.1 | 4.9 ± 1.1^{c,d} | 5.9 ± 1.7^{e} |

| | | | |
|---|---|---|---|
| Differences in age: ^{a} RR-PP, p=0.003; ^{b} controls-PP, p=0.017 | | | |
| Differences in EDSS: ^{c} RR-SP, p≤0.0001; ^{d} SP-PP, p=0.014; ^{e} RR-PP, p≤0.0001 | | | |

### mRNA isolation and cDNA synthesis

### See Experimental part I

### Non-competitive RT-PCR

cDNA derived from whole blood mRNA of the patients was quantified in a semiquantitative fashion. To this end the cDNA was amplified in triplicate. Quantification was enabled employing simultaneous amplification of step-fold diluted external standards. Precaution was taken that all samples were in the log-linear range of amplification as defined by the amplified standards. Unless indicated otherwise the multi-specific vectors pQA-1 and pQB-3 (kindly provided by Drs. D. Shire and P. Legoux, Sanofi Recherche, Labège Cedex, France) were used. For IL-12p40 and IL-12p35, a pQA-1/IL-12 construct prepared as described previously was used. A standard for TGF-β 1 was kindly provided by H. Baelde and Dr. E. de Heer (Dept. of Pathology, Leiden University Medical Center, The Netherlands). To enable quantification of IL-18, cDNA was prepared using RNA derived from PBMC (obtained from a healthy donor) that were stimulated for 4 hours with 250 ng/ml LPS (*Escherichia coli-derived*, serotype 0127:B8, Sigma, St Louis, MO). To enable quantification of IL-12Rβ1 and IL-12Rβ2, cDNA was prepared by using RNA derived from PBMC that were stimulated for 3 days with 1 µg/ml PHA (HA 16, Phaseolus-species; Murex Biotech LTD, Dartford, Kent, England). Table 5 shows the sequence of the primers used for the amplification of IL-4, TGF-β1, IL-18, IL-12Rβ1 and IL-12Rβ2 and the size of the amplified products of cell-derived cDNA and standard-DNA (for IL-4 and TGF-β1 only).

**Table 5:**

| Primer sequences used and size of corresponding amplicons | | | |
|---|---|---|---|
| | | cDNA | standard |
| TGF-β sense | AGCGACTCGCCAGAGTGGTTATCTT | 473bp | 600bp |
| TGF-β antisense | TTATGCTGGTTGTACAGGGCCAGGA | | |
| IL-4 sense | TGCCTCCAAGAACACAACTG | 224bp | 370bp |
| IL-4 antisense | AACGTACTCTGGTTGGCTTC | | |
| IL-18 sense | GCTTGAATCTAAATTATCAGTC | 342bp | |
| IL-18 antisense | GAAGATTCAAATTGCATCTTAT | | |
| IL-12Rβ1 sense | CCTGAAAACCCCCCACAG | 637 bp | |
| IL-12Rβ1 antisense | GCTGAGGCATTGCCCCCA | | |
| IL-12Rβ2 sense | CTGCAAACTGGCCTGTATCAA | 430bp | |
| IL-12Rβ2 antisense | GTGCTCTCAATGATTCACTCC | | |

Primer sequences used for amplification of the other cDNA products have been published previously (Visser J, van Boxel-Dezaire A, Methorst D, Brunt T, de Kloet ER, Nagelkerken L., Blood 1998;91:4255-4264; and van Boxel-Dezaire et al., Ann. Neurol. 1999;45:695-703). Using a Perkin Elmer 9600 PCR apparatus, PCR reactions with PQA-1, PQB-3, TGF-β1, IL-18 or IL-12Rβ chain-primers were performed in PCR buffer I (10 mM Tris/HCl, pH 8.3, containing 50 mM KCl, 2 mM MgCl₂, 60 µg/ml BSA), in the presence of 0.25 mM dNTPs (Gibco BRL, Gaithersburg, MD), 12.5 pmol sense and antisense primers (Isogen Bioscience, Maarssen, The Netherlands) and 1 unit Taq DNA polymerase (Gibco BRL) in a final volume of 50 µl. Similar conditions were used for the amplification of PQA-1/IL-12 with the exception that PCR buffer I was replaced by PCR buffer II (50 mM KCl, 10 mM Tris/HCl pH 8.3, 100 µg/ml gelatin and 2.5 mM MgCl₂; Perkin Elmer). After 2 min of denaturation at 96 °C, the following cycling conditions were used: 0.5 min of denaturation at 96 °C, 1 min annealing at 55 °C (60 °C for IL-12Rβ1 and β2 amplification) and 1 min elongation at 72 °C ; these steps were repeated during 30 cycles for β-actin and during 35 cycles for the cytokines and receptors. These reactions were terminated with an elongation step of 4 min at 72 °C.

IL-4 and TGF-β1 were exceptional in that, after 2 min of denaturation at 96 °C, the following cycling conditions were used: 0.5 min of denaturation at 96 °C and 1 min annealing at the following temperatures. During the first two cycles, the annealing temperature was 58 °C (65 °C for TGF-β1); subsequently this temperature was decreased with 1 °C after every two cycles until 54 °C (61 °C for TGF-β1) was reached, followed by ten cycles at 53°C (60 °C for TGF-β1) and twenty cycles at 52 °C (59 °C for TGF-β1). The PCR products for β-actin, TNF-α, IL-18 and TGF-β1 were separated on a 2 % agarose gel and stained with ethidium bromide. Visualization of the other PCR products required enhancement employing SYBR Green I nucleic acid gel stain (Molecular Probes Europe BV, Leiden, The Netherlands). The amount of PCR product was determined by densitometric imaging using the Bio-lD digital imaging system version 6 (Vilber Lourmat, Marne la Vallée, France). The amounts of cytokine (receptor) cDNA were calculated with the use of the external calibration curves and expressed as fg standard equivalents (or arbitrary units for IL-18, IL-12Rβ1 and β2). To correct for differences in initial cell numbers, as well as the efficiency of mRNA isolation and cDNA synthesis, all values were normalized by calculating the ratio of cytokine (receptor) to β-actin and multiplied with 10⁶.

### Statistical Methods

Statistical analysis was performed with the use of the SPSS 8.0 package (SPSS Inc, Chicago, IL) for MS-Windows. The Chi-Square test was used to detect whether differences existed in the female/male distribution between the studied controls, RR, SP and PP patients; this appeared not to be the case. The Kruskal-Wallis test was performed to establish whether differences existed between RR, SP and PP MS patients regarding disease duration and EDSS. Furthermore, this analysis was done to establish differences between healthy controls, RR, SP and PP patients with respect to age. The Kruskal-Wallis test showed significant differences in EDSS (p≤0.0001) and age (p=0.017) only. Subsequently, the Mann-Whitney U-test was used to establish which groups were different in EDSS and age (Table 4).

In order to establish a normal distribution of the data, the cytokine (receptor) mRNA data were log-transformed and used during all analyses except the Spearman correlation test. ANOVA was used to identify possible differences in mRNA expression levels between the 4 groups (controls, RR, SP and PP). Only when this analysis showed significant differences between the groups (p<0.05), the least significant differences (LSD) method was used to analyze precisely which groups differed from each other. The Spearman correlation test was performed to analyze whether the cytokine (receptor) mRNA data were correlated with EDSS (within each individual group). To establish whether certain parameters were distinctive for a clinical subgroup of MS, a discriminant analysis was performed including all cytokine (receptor) mRNA data. The error rates of the classification rule were established by cross-validation. All data shown are the percentages after cross-validation (Table 7).

### Results

### Differences in cytokine and IL-12 receptor mRNA levels expressed in whole blood of healthy controls and patients with different clinical subtypes of MS

Cytokine (receptor) mRNA levels expressed in whole blood cells of healthy controls, RR-, SP- and PP-MS were studied; sixteen persons were included in each group in order to establish differences in the clinical subtypes of MS. Figure 3 shows the geometric means with their 95% confidence interval for each immunological parameter measured in each group. First, statistical analysis was performed including all data points in order to identify possible differences in mRNA expression levels between the 4 groups. This analysis demonstrated significant differences between the 4 groups in the anti-inflammatory cytokines IL-10 (p=0.001), TGF-β (p≤0.0001) and IL-4 (p=0.023) and in the pro-inflammatory cytokines IFN-γ (p≤0.0001), IL-12p40 (p≤0.0001) and IL-18 (p≤0.0001). Moreover, this analysis showed significant changes in the two chains that together form a functional IL-12R (IL-12Rβ1 and IL-12Rβ2: both p≤0.0001). To establish which groups differed significantly in mRNA expression, further statistical analysis was performed on all measured parameters, except IL-12p35 and TNF-α mRNA.

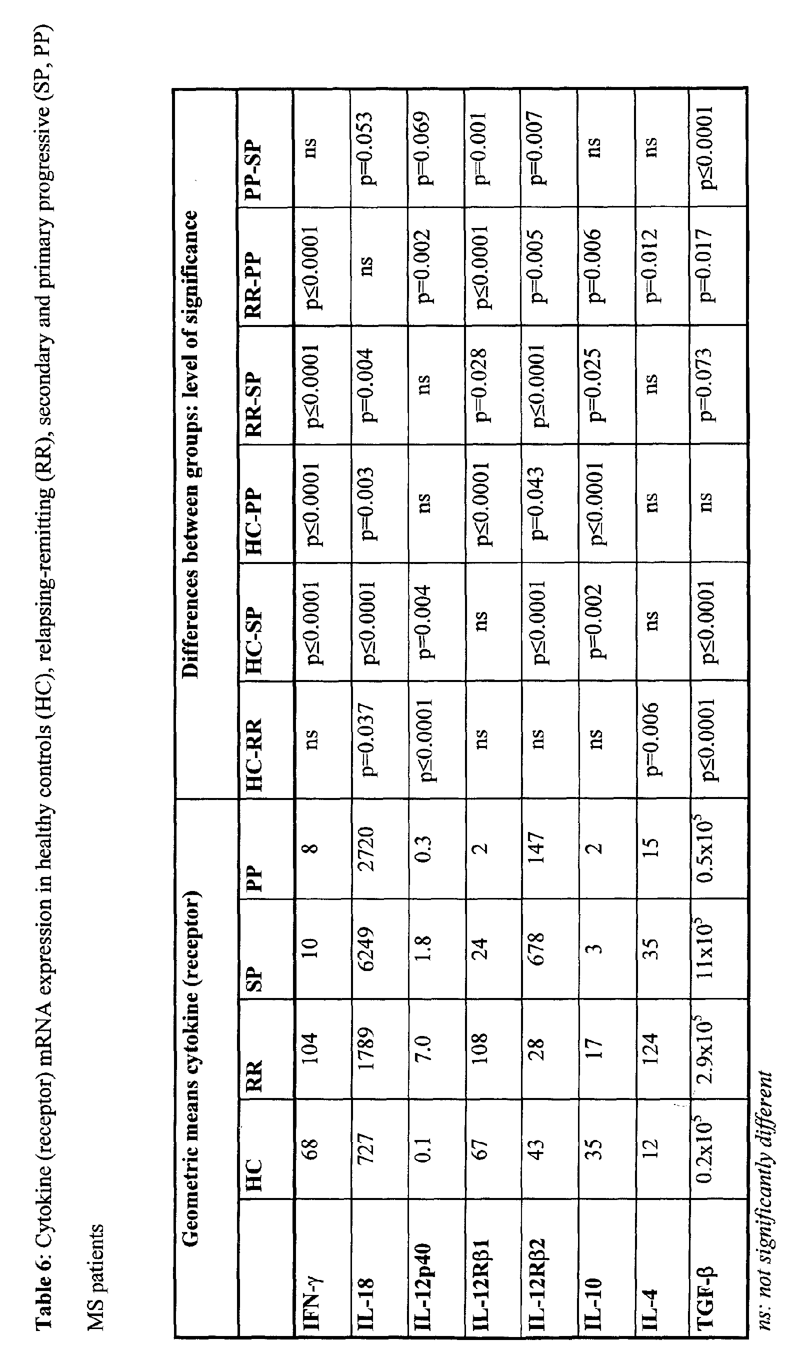

### Differences between healthy controls, RR and SP MS patients

After having established in a general analysis which cytokine (receptor) mRNAs showed differences, a more detailed analysis was employed to identify which groups were different. Increased levels of both IL-18 and IL-12p40 mRNA were demonstrated in RR MS compared to healthy controls (Table 6). Moreover, these patients also showed higher levels of TGF-β and IL-4 mRNA.

Differences with healthy controls were even more pronounced in the secondary progressive phase of MS (Table 6). These patients showed increased TGF-β, IL-18, IL-12p40 and IL-12Rβ2 mRNA and decreased IL-10 and IFN-γ mRNA as compared to the controls. As compared to the relapsing-remitting phase, the secondary progressive phase was characterized by decreased levels of IL-10 and IL-12Rβ1 (4-fold) mRNA. The IL-12Rβ2 chain was strongly increased (24-fold) compared to the RR patients. Unexpectedly, we found that IFN-γ mRNA was strongly decreased as compared to controls and RR patients.

### Differences between RR, SP MS patients and PP MS patients

It is still unclear to what extent the immune system is involved in the pathogenesis of PP MS. Therefore we first compared immunological parameters of these patients with healthy controls and RR patients. As shown in Table 6, there was only little similarity between the cytokine (receptor) mRNA expression patterns found in PP and RR MS patients. However, both subgroups had in common that IL-18 mRNA levels were increased compared to healthy controls. As opposed to RR patients, PP patients did not show increased IL-12p40, TGF-β and IL-4 mRNA levels compared to controls; therefore these levels were significantly higher in RR as compared to PP MS. The primary progressive phase was associated with lower IL-10 and IFN-γ levels as compared to RR patients and controls. Interestingly, IL-12Rβ2 mRNA levels were enhanced in PP patients compared to RR patients (5-fold) and controls (3-fold), whereas IL-12Rβ1 mRNA levels were strongly decreased (44-fold and 27-fold, respectively).

Next, patients in the primary and secondary progressive phase of MS were compared (Table 6). Statistical analysis showed similarities, but also differences in mRNA expression between SP and PP patients. Both SP and PP patients had increased IL-18 mRNA and decreased IL-10 and IFN-γ mRNA levels as compared to the controls. However, highest IL-18 mRNA levels were found in the SP group. IL-12p40 and TGF-β mRNA levels in PP patients were equal to those in the controls; therefore, differences in these cytokine mRNAs between PP patients and SP patients were due to increased levels in the latter group. Marked differences were also found for both the IL-12R chains. Levels of IL-12Rβ1 mRNA were tenfold lower in PP patients compared to SP patients. Although both PP and SP MS patients had increased IL-12Rβ2 levels as compared to controls, the levels of this chain were fivefold higher in SP patients as compared to PP patients. From these data it can be concluded that blood cells from PP patients display an activation state that is different from healthy controls, RR and SP patients.

The data shown in Table 6 are suggestive for extensive immune activation in MS. Moreover, these data suggest that cells of the immune system are differently activated in the patient subgroups. In order to visualize which patterns existed in the MS patients, the fold change with respect to the geometric mean of the healthy controls was calculated for each cytokine (receptor) expressed in each MS patient group (Figure 4). Cytokine (receptor) mRNA expression levels, which were on average 10 times higher or lower than the levels of the controls (p<0.01) were considered of importance. It is evident from this figure that only RR and SP patients show increased pro-inflammatory cytokine levels. Only SP patients showed increased IL-18 and IL-12Rβ2 mRNA levels. PP patients are unique in that they showed decreased IL-12Rβ1 mRNA levels. RR patients show increased IL-4 and normal IL-10 mRNA levels, whereas secondary and primary progressive patients show normal IL-4 and decreased IL-10 mRNA levels.

### PP patients do not show an inverse correlation between IL-10 and EDSS

In an earlier study we showed that decreased IL-10 mRNA levels were correlated with MRI-activity in RR and SP patients. In this study, we investigated whether a correlation existed between this cytokine and the EDSS-score in any of the MS patient groups. The RR (p= -0.400, p=0.125) and PP (p = 0.384, p=0.157) patients did not show a significant correlation between IL-10 and increasing EDSS, whereas the SP patients did (p = -0.529, p=0.035). This suggests that disease progression was only associated with declining levels of IL-10 in the SP patients. Moreover, only SP patients showed a positive correlation between increasing EDSS and increasing levels of IL-12Rβ1 (p = 0.605, p=0.013). For neither the RR nor the PP patients a significant correlation could be found between the EDSS-score and any of the measured immunological markers.

### Cytokine (receptor) mRNA discriminates between clinical phases of MS

Discriminant analysis was performed to analyze which immunological parameters were most discriminative between the different groups. It appeared that IL-18, IL-12p40 and IL-12Rβ1 mRNA levels correctly assigned 91.3% of the patients to the MS group; 83.3% of the healthy controls were correctly assigned to the control group (data not shown).

Next, discriminant analysis was done comparing healthy controls, RR and PP patients, because this may help establishing the diagnosis MS (Table 7A). When 3 groups (i.e. controls, RR and PP MS) have to be classified, 2 discriminant functions are needed, combining the best discriminative parameters (Table 7A). For each tested individual the outcome of such a function was calculated and subsequently the means were determined for the different groups of MS patients and controls (Table 7A). The statistical analysis calculates the shortest distance to a mean of a group and this determines to what group an individual is assigned. It appeared that with IFN-γ, IL-18, IL-12Rβ1 and IL-12Rβ2 mRNA a distinction was possible between PP patients and controls or RR patients (Table 7A). Furthermore, IL-12p40 and IL-18 mRNA were most distinctive between controls and RR patients. With these levels, it is possible to classify on average 83% of the controls, RR and PP patients correctly (75.0, 86.7 and 86.7 % respectively, Table 7A).

It is of great importance to know at which moment RR patients become progressive in their disease. Discriminant analysis showed that on average 97% of the RR and SP patients could be correctly classified using IFN-γ, IL-10 and IL-12Rβ2 mRNA levels (Table 7A; 100 and 93.8% respectively). Furthermore, it is important to test if it is possible to discriminate between secondary and primary progressive MS patients. This analysis showed that on average 75% of the SP and PP patients could be correctly classified with the mRNA levels of TGF-β (Table 7A).

**Table 7A:**

| Cytokine (receptor) mRNA discriminates between controls and clinical subtypes in MS | | | |
|---|---|---|---|
| **Comparison** | **Discriminant function** | **Correctly classified** | |
| **HC, RR and PP** | **F1=** -0.104 + 0.575^{*}lnIFN-γ + 0.552^{*}lnIL-12Rβ1 - | HC: 75.0 % | |
| | 0.349^{*}lnIL-12Rβ2 - 0.309^{*}lnIL-18 | RR: 86.7% | |
| | | PP: 86.7% | |
| | Outcome: | Mean: 83.0% | |
| | mean HC= 1.36; mean RR= 1.50; mean PP=-2.59 | | |
| | **F2=** -2.401 +0.525*InIL-18 + 0.331*InIL-12p40 | | |
| | Outcome: | | |
| | mean HC= -1.22; mean RR= 0.94 | | |
| **RR and SP** | **F3=** 0.911 - 0.607*InIL-12Rβ2 + 0.422*InIFN-γ + | RR: 100.0% | |
| | 0.309^{*}lnIL-10 | SP: 93.8% | |
| | | Mean: 96.8% | |
| | Outcome: | | |
| | mean RR= 1.83; mean SP= -1.72 | | |
| **SP and PP** | **F4=** -6.555 + 0.530*InTGF-β | SP: 75.0% | |
| | | PP: 75.0% | |
| | Outcome: | Mean: 75.0% | |
| | mean SP= 0.82; mean PP= -0.82 | | |

Finally, discriminant analysis was performed to identify which of the cytokines or IL-12 receptor chains were most discriminative between the different MS subgroups. It appeared that with IFN-γ, IL-12Rβ2 and TGF-β mRNA on average 78% of the MS patients were correctly classified to the different MS subtypes (Figure 5 and Table 7B). IFN-γ and IL-12Rβ2 mRNA levels discriminated the RR patients from the progressive patients (F1, Table 7B). Moreover, TGF-β mRNA levels distinguished the SP from the PP patients (F2, Table 7B). When a new unclassified person has to be classified to a certain phase of MS, the outcomes of above mentioned mathematical functions are useful to determine to which group an individual can be assigned.

**Table 7B:**

| Cytokine (receptor) mRNA discriminates between clinical subtypes in MS | | | | |
|---|---|---|---|---|
| **Discrimant functions** | | | | |
| | | **Means (st.dev =1 by construction)** | | |
| **Function** | **Definition** | **RR** | **SP** | **PP** |
| F1 | -2.156 + 0.559*lnIFN-γ-0.538*lnIL-12Rβ2 | 1.84 | -0.84 | -0.95 |
| F2 | -6.317 + 0.501^{*}lnTGF-β | -0.03 | 0.86 | -0.88 |

| **Classification results obtained via cross-validation** | | | | |
|---|---|---|---|---|
| | | **Classified as** | | |
| | | **RR (%)** | **SP (%)** | **PP (%)** |
| **True subtype** | **RR** | 93.3 | 0.0 | 6.7 |
| | **SP** | 6.3 | 75.0 | 18.8 |
| | **PP** | 6.7 | 26.7 | 66.7 |

MS is characterized by a rather unpredictable disease progression in an individual patient. On the basis of an international survey, consensus has been reached to divide MS in 4 different clinical subgroups: relapsing-remitting MS, secondary progressive MS, primary progressive MS, and progressive-relapsing MS. Possibly different pathological mechanisms are responsible for these different clinical appearances. About 85% of the MS patients are characterized by an onset with a RR pattern of which eventually more than 50% develop a SP pattern. Since optimal treatment may be quite different for the two phases, it is of importance to identify markers that predict the transition into the secondary phase. Inflammatory lesions that can be visualized by MRI using gadolinium-enhancement characterize both the RR and the SP stage. Recently, we showed that both in RR and SP patients the occurrence of active lesions is preceded by low levels of IL-10 and high levels of IL-12p40 mRNA. In the present study, both patient groups showed increased levels of IL-12p40 mRNA and as a result the IL-12p40/IL-10 ratio was 125-fold higher in RR patients and 181-fold higher in SP patients as compared to the controls (both p≤0.0001, data not shown). In addition, both groups of patients and in particular SP patients showed increased IL-18 and TGF-β mRNA levels, suggesting an increased state of activation of monocytes. TGF-β, in general thought to posses anti-inflammatory properties, has in common with IL-12 and IL-18 that naive T cells are skewed towards the Th1 subset which is characterized by expression of IL-12Rβ2 mRNA. Furthermore, IL-12 and IL-18 secreted by antigen presenting cells are able to upregulate IL-12Rβ2 mRNA in established Th1 cells, whereas IL-10 downregulates this expression. The fact that we only found increased IL-12Rβ2 mRNA levels in the SP patients may be explained by higher levels of IL-18 mRNA in these patients as compared to the RR patients, or by lower IL-10 levels. In accordance, we found that IL-18 and the IL-12Rβ2 mRNA were positively correlated in SP patients (p=0.709, p=0.002). In contrast, we did not observe an inverse correlation between IL-10 and IL-12Rβ2.

The expression of both the IL-12Rβ1 and IL-12Rβ2 chains are necessary for a functional IL-12R. Although the IL-12Rβ1 mRNA levels were fourfold lower in SP compared to RR MS, the IL-12Rβ2 mRNA levels were 24-fold higher in SP patients. It is likely that this results in increased expression levels of the IL-12R and increased sensitivity to IL-12 in SP compared to RR MS. This is in agreement with the findings of Becher and colleagues who used intracellular staining of IFN-γ in CD4⁺ T cells to demonstrate that increased numbers of Th1 cells exist in the SP patients, but not in the RR phase. In our cross-sectional study as well as our earlier published longitudinal study we found decreased IFN-γ mRNA levels in the SP patients, which is not expected in view of the increased IL-12Rβ2 mRNA levels found here. Decreased levels of IFN-γ mRNA in NK cells or an unique T cell subset may explain these findings. An alternative explanation may be the fact that TGF-β is able to downregulate IFN-γ production via the inhibition of IL-12 signaling, without affecting IL-12Rβ1 and IL-12Rβ2 mRNA expression.

In relation to MRI lesions, we have already shown differences between RR and SP patients with regard to the control of disease activity. Whereas SP patients showed constitutively low levels of IL-10 mRNA before, during and after the development of MRI lesions, RR patients showed rather normal IL-10 mRNA levels during MRI activity and the remission phase. In this cross-sectional study we confirm that levels of IL-10 mRNA are decreased in SP compared to RR patients. In addition, the SP patients did not show enhanced levels of IL-4 mRNA, suggesting that the SP patients are less able to control progression of disease by the upregulation of anti-inflammatory cytokines. This is supported by the finding that only in the SP patients IL-10 mRNA was inversely correlated with EDSS, suggesting that progression of disease in this patient group was associated with declining levels of IL-10. In conclusion, these data show that increased levels of pro-inflammatory cytokines are both found in RR and SP patients, whereas lower amounts of anti-inflammatory cytokines are found in SP patients in particular. Furthermore, discriminant analysis showed that decreased IL-10 and IFN-γ and increased IL-12Rβ2 mRNA levels were indicative for the progression into the secondary progressive phase. It is tempting to speculate that this is due to the depletion of Tr1 cells, which are known to produce these cytokines simultaneously.

Little is known about the involvement of the immune system in PP MS. The view that the immune system is not involved in the pathogenesis of PP patients has recently been challenged, because despite of the absence of gadolinium enhancement on MRI these patients showed blood-brain barrier impairment and enhanced levels of soluble E-selectin. Herein we show for the first time that unstimulated whole blood cells of PP patients display a different activation pattern compared to healthy controls and SP patients. Moreover, the cytokine (receptor) expression pattern was quite different in PP patients compared to RR patients, suggesting that different pathological mechanisms are involved in the onset and progression of disease. Remarkably, PP patients not only showed lower pro-inflammatory cytokine expression but also lower IL-12Rβ levels compared to RR (44-fold) and SP (10-fold) patients. Because IL-12Rβ2 mRNA levels were also lower (5-fold) in PP patients compared to SP patients, it is likely that IL-12R levels are lower than in SP patients. These data are suggestive for less involvement of pro-inflammatory compounds in the pathogenesis of PP MS. This is in line with the finding that PP patients show significantly less inflammatory lesions than SP patients, based on the frequency of perivascular cuffing and cellularity of the parenchyma. Thus, the generally accepted idea that macrophages are stimulated to become involved in demyelination of axons in the central nervous system after their activation by myelin-specific Th1 cells, may only apply for RR and SP patients. Others have already suggested different pathological mechanisms in SP and PP patients, because primary oligodendrocyte destruction was demonstrated in lesions of autopsy material from PP patients, whereas demyelination seemed to be a secondary phenomenon. Furthermore, PP patients show a much more pronounced loss in cord cross-sectional area during 12 months of follow-up than SP patients. In SP patients as well as RR patients, we showed increased IL-12p40 mRNA levels before the development of active lesions. The observation that PP patients show much less active lesions on brain and spinal cord MRI than RR and SP patients, may hence be explained by the lower IL-12p40 and IL-12R levels in PP MS. This is the first study postulating an immunological explanation for less frequent occurrence of active lesions in PP MS than in RR and SP MS.

Interestingly, primary progressive patients shared with secondary progressive patients a decrease in IFN-γ and IL-10 mRNA. Since declining IL-10 levels seem to be involved in the progression of disease from RR into the SP phase, low IL-10 levels in the PP patients may explain why disease in these patients is progressive from onset on. However, IL-10 mRNA levels were not inversely correlated with the EDSS-score in PP patients, as was the case in SP patients, suggesting the lack of involvement of IL-10 in disease progression in PP. This suggests that a different mechanism is responsible for disease progression in PP patients. Lower IFN-γ mRNA levels in PP patients may be due to lower levels of functional IL-12 receptor and are possibly a reflection of lower numbers of Th1 cells. It can not be excluded that pro-inflammatory cytokines even prevent progression of disease and have a protective function in PP patients. The possibility that pro-inflammatory cytokines are able to prevent disease progression has been described in an EAE-model, were antibody mediated disease was inhibited by TNF-α. Antibodies may be involved in the pathogenesis of PP MS, because increased levels of anti-ganglioside antibodies have been found; these may be the factors in the CFS of PP patients that induce apoptosis of neurons. Further research is needed to elucidate the exact involvement of the immune system in the pathogenesis of PP patients. Comparisons of measurements of above mentioned cytokines as expressed in T cell subsets and monocytes of RR, SP and PP patients may provide further clues. Recently, problems were shown in designing and recruiting therapeutic trials in PP MS, because of the unsecure diagnosis. Measuring cytokine (receptor) mRNA shows great promise in solving these problems, since with these levels it is possible to distinguish RR and PP MS patients from healthy controls and RR and SP from PP patients. However, these findings need to be confirmed in another independent study.

In conclusion, from the parameters under investigation and by thorough statistical analysis IFN-γ, IL-12p40, IL-12Rβ1, IL-12Rβ2, IL-18, IL-10 and TGF-β mRNA levels appear valuable as diagnostic tools for the discrimination between healthy controls, RR, SP and PP MS patients.

## Claims

1. Method for following the progres and/or treatment of multiple sclerosis on the basis of at least one biological sample obtained from a person suffering from, or suspected to suffer from, multiple sclerosis, said method comprising the steps of:
a) providing a biological sample derived from a patient suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1, IL-12Rβ2 and/or IL-12p35 of said first biological sample; and optionally
c) comparing for one or more of the cytokines determined in step b) the value(s) obtained in step b) with a reference value.

2. Method for determing the success rate of treatment of multiple sclerosis by discriminating between patients with multiple sclerosis (regardless of the clinical subtype) and healthy controls, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

3. Method according to claim 2, in which, in step b), one or more of the group consisting of IFN-γ, IL-12p40, IL-12Rβ1, IL-12Rβ2 and IL-18 is determined.

4. Method according to claim 2 and/or 3, in which, in step b), at least IL-18 is determined.

5. Method for determing the success rate of treatment of multiple sclerosis by discriminating between the different subtypes of multiple sclerosis, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

6. Method according to claim 5, in which, in step b) one or more of the group consisting of TGF-β, IFN-γ, IL-10, IL-12Rβ1 and/or IL-12Rβ2 is determined.

7. Method according to claim 5 and/or 6, in which, in step b), at least one or more of the group consisting of TGF-β, IFN-γ, and/or IL-12Rβ2 is determined.

8. Method for determing the success rate of treatment of multiple sclerosis by discriminating between relapsing remitting multiple sclerosis and secondary progressive multiple sclerosis, on the basis of at least one biological sample obtained from a person suffering from, or suspected to suffer from, multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IL-18, IFN-γ, IL-10, IL-12Rβ1 and/or IL-12Rβ2; and optionally
c) comparing, for one or more of the cytokines determined in step b), the value(s) obtained in step b) with at least one reference value, e.g. for the same cytokine.

9. Method according to claim 8, in which, in step b), one or more of the group consisting of IL-12Rβ2, IFN-γ and IL-10 is determined.

10. Method according to claim 8 and/or 9, in which, in step b), at least IL-12Rβ2 is determined.

11. Method for determing the success rate of treatment of multiple sclerosis by discriminating between primary progressive multiple sclerosis and secondary progressive multiple sclerosis, on the basis of at least one biological sample obtained from a person suffering from or suspected to suffer from multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IL-12p40, IL-18, TGF-β, IL-12Rβ1 and/or IL-12Rβ2; and optionally
c) comparing, for one or more of the cytokines determined in step b), the value obtained in step b) with at least one reference value, e.g. for the same cytokine.

12. Method according to claim 11, in which, in step b), one or more of the group consisting of TGF-β, IL-12Rβ1 and IL-12Rβ2 is determined.

13. Method according to claim 11 and/or 12, in which, in step b), at least TGF-β is determined.

14. Method for determing the success rate of treatment of multiple sclerosis by discriminating between remitting relapsing multiple sclerosis and primary progressive multiple sclerosis, on the basis of at least one biological sample obtained from a person suffering from or suspected to suffer from multiple sclerosis, said method comprising the steps of:
a) providing at least one biological sample derived from said person suffering from, or suspected to suffer from, multiple sclerosis;
b) determining in said at least one biological sample the amount of at least one of the cytokines from the group consisting of IFN-γ, IL-12p40, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

15. Method according to claim 14, in which, in step b), one or more of the group consisting of IL-12Rβ1, IL-12Rβ2 and IFN-γ is determined.

16. Method according to claim 14 and/or 15, in which, in step b), at least IFN-γ is determined.

17. Method for determing the success rate of treatment of multiple sclerosis by determining whether a biological sample (or a set of biological samples) has been derived from a patient with multiple sclerosis (regardless of the clinical subtype) or from a healthy control, said method generally comprising the steps of:
a) providing said biological sample(s) from a patient suffering from, or suspected to suffer from, multiple sclerosis;
b) determining in said biological sample(s) the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

18. Method according to claim 17, in which, in step b), one or more of the group consisting of IFN-γ, IL-12p40, IL-12Rβ1, IL-12Rβ2 and IL-18 is determined.

19. Method according to claim 17 and/or 18, in which, in step b), at least IL-18 mRNA is determined.

20. Method for determing the success rate of treatment of multiple sclerosis by determining whether a biological sample (or a set of biological samples) has been derived from a patient with multiple sclerosis, and if so, whether said (set of) biological sample(s) has been derived from a patient suffering from relapsing remitting multiple sclerosis, secondary progressive multiple sclerosis and/or primary progressive multiple sclerosis, said method generally comprising the steps of:
a) providing said biological sample(s) from a patient suffering from, or suspected to suffer from, multiple sclerosis;
b) determining in said biological sample(s) the amount of at least one of the cytokines from the group consisting of IL-18, IL-12p40, IFN-γ, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

21. Method according to claim 20, in which, in step b), one or more of the group consisting of TGF-β, IFN-γ, IL-10, IL-12Rβ1 and/or IL-12Rβ2 are determined.

22. Method according to claim 20 and/or 21, in which, in step b), at least one or more of the group consisting of TGF-β, IFN-γ, and/or IL-12Rβ2 are determined.

23. Method for determing the success rate of treatment of multiple sclerosis by determining whether a biological sample (or a set of biological samples) has been derived from a patient with relapsing remitting multiple sclerosis or from a patient with secondary progressive multiple sclerosis, said method generally comprising the steps of:
a) providing said biological sample(s) from a patient suffering from, or suspected to suffer from, multiple sclerosis, and in particular from a patient suspected to suffer from either relapsing remitting multiple sclerosis or secondary progressive multiple sclerosis;
b) determining in said biological sample(s) the amount of at least one of the cytokines from the group consisting IL-18, IFN-γ, IL-10, IL-12Rβ1 and/or IL-12Rβ2; and optionally
c) comparing, for one or more of the cytokines determined in step b), the value(s) obtained in step b) with at least one reference value, e.g. for the same cytokine.

24. Method according to claim 23, in which, in step b), one or more of the group consisting of IL-12Rβ2, IFN-γ and IL-10 is determined.

25. Method according to claim 23 and/or 24, in which, in step b), at least IL-12Rβ2 is determined.

26. Method for determing the success rate of treatment of multiple sclerosis by determining whether a biological sample (or a set of biological samples) has been derived from a patient with primary progressive multiple sclerosis or from a patient with secondary progressive multiple sclerosis, said method generally comprising the steps of:
a) providing said biological sample(s) from a patient suffering from, or suspected to suffer from, multiple sclerosis, and in particular from a patient suspected to suffer from either primary progressive multiple sclerosis or secondary progressive multiple sclerosis;
b) determining in said biological sample(s) the amount of at least one of the cytokines from the group consisting of IL-12p40, IL-18, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

27. Method according to claim 26, in which, in step b), one or more of the group consisting of TGF-β, IL-12Rβ1 and IL-12Rβ2 is determined.

28. Method according to claim 26 and/or 27, in which, in step b), at least TGF-β is determined.

29. Method for determing the success rate of treatment of multiple sclerosis by determining whether a biological sample (or a set of biological samples) has been derived from a patient with relapsing remitting multiple sclerosis or from a patient with primary progressive multiple sclerosis, said method generally comprising the steps of:
a) providing said biological sample(s) from a patient suffering from, or suspected to suffer from, multiple sclerosis, and in particular from a patient suspected to suffer from either relapsing remitting multiple sclerosis or primary progressive multiple sclerosis
b) determining in said biological sample(s) the amount of at least one of the cytokines from the group consisting of IFN-γ, IL-12p40, IL-4, IL-10, TGF-β, IL-12Rβ1 and/or IL-12Rβ2; and optionally
c) comparing, for one or more of the cytokines determined in step b), the value(s) obtained in step b) with at least one reference value, e.g. for the same cytokine.

30. Method according to claim 29, in which, in step b), one or more of the group consisting of IL-12Rβ1, IL-12Rβ2 and IFN-γ is determined.

31. Method according to claim 29 and/or 30, in which, in step b), at least IFN-γ is determined.

32. Method for determining the success rate of treatment of multiple sclerosis, and in particular of treatment of multiple sclerosis with interferon-beta (IFN-β) species such as IFN-β1b and IFN-β1a, said method comprising the steps of:
a) providing a biological sample derived from a patient suffering from, or suspected to suffer from, multiple sclerosis;
b) determining the amount of at least one of the cytokines from the group consisting of IL-12p35, IL-18 and TGF-β of said first biological sample;
c) optionally comparing the value obtained in step b) with a reference value.

33. Method according to claim 32, in which in step b) either the amount of IL-12p35, of IL-18 and/or of TGF-β, the amount of any two thereof, and/or the amount of all three thereof is determined.

34. Method according to claim 32 and/or 33, in which in step b) at least the amount of IL-12p35 is determined.

35. Method for determining the success rate of treatment of multiple sclerosis, and in particular of treatment of multiple sclerosis with interferon-beta (IFN-β) species such as IFN-β1b and IFN-β1a, said method comprising the steps of:
a) providing a first biological sample derived from a patient suffering from, or suspected to suffer from, multiple sclerosis;
b) determining at least the amount of IL-10 of said first biological sample;
c) providing a second biological sample from the patient of step a), after said patient has been treated with interferon-β for a period of 1 to 20 days, or 20 to 40 days, or 40 to 60 days, or 60 to 80 days, preferably for a period of 20 to 40 days, more preferably for 30 days;
d) determining at least the amount of IL-10 of second biological sample;
e) optionally comparing the value measured during step b) with the value measured during step d).

36. Method for determining the success rate of treatment of multiple sclerosis, and in particular of treatment of multiple sclerosis with interferon-beta (IFN-β) species such as IFN-β1b and IFN-β1a, said method comprising the steps of:
a) providing a first set of one or more biological sample(s) derived from a patient suffering from, or suspected to suffer from, multiple sclerosis;
b) determining at least the amount of IL-10 in at least one sample from the first set obtained in step a); and also determining the amount of at least one of the cytokines chosen from the group IL-12p35, IL-18 and TGF-β in the same or a different sample from the first set obtained in step a);
c) optionally comparing the value obtained in step b) for IL-12p35, IL-18 and TGF-β with a reference value;
d) providing a second set of one or more biological sample(s) from the patient of step a), after said patient has been treated with interferon-β for a period of 1 to 20 days, or 20 to 40 days, or 40 to 60 days, or 60 to 80 days, preferably for a period of 20 to 40 days, more preferably for 30 days;
e) determining at least the amount of IL-10 in at least one sample of the second set obtained in d);
f) optionally comparing the value(s) measured for IL 10 during step b) with the value(s) measured during step e).

37. Method according to claim 36, in which in step b) also either the amount of IL-12p35, of IL-18 and/or of TGF-β, the amount of any two thereof, and/or the amount of all three thereof are determined.

38. Method according to claim 36 or 37, in which in step b) also at least the amount IL-12p35 is determined.

39. Method according to claim 36, in which in step b) and in step d) also either the amount of IL-12p35, of IL-18 and/or of TGF-β, the amount of any two thereof, and/or the amount of all three thereof are determined

40. Method according to claim 39, in which in step b) and in step d) also at least the amount of IL-12p35 is determined.

41. Method according to any of the preceding claims, in which the amount of cytokine is determined by determining the amount of mRNA encoding said cytokine present in the biological sample.

42. Method for determining the success rate of treatment of multiple sclerosis, and in particular of treatment of multiple sclerosis with interferon-beta (IFN-β) species such as IFN-β1b and IFN-β1a, said method comprising the steps of:
a) providing a first biological sample derived from a patient suffering from, or suspected to suffer from, multiple sclerosis;
b) determining at least the amount of IL-10 protein in CD4⁺ Tcells of said first biological sample;
c) providing a second biological sample from the patient of step a), after said patient has been treated with interferon-β for a period of 0 to 50 days, or 50 to 100 days, or 100 to 150 days, or 150 to 200 days, preferably for a period of 150 to 200 days, more preferably for 180 days;
d) determining at least the amount of IL-10 protein in CD4⁺ T cells of second biological sample;
e) optionally comparing the value measured during step b) with the value measured during step d).

43. Method for determining the success rate of treatment of multiple sclerosis, and in particular of treatment of multiple sclerosis with interferon-beta (IFN-β) species such as IFN-β1b and IFN-β1a wherein one or more methods according to any of the claims 35-41 is used in combination with the method according to claim 42.

44. Method for determining the success rate of treatment of multiple sclerosis, and in particular of treatment of multiple sclerosis with interferon-beta (IFN-β) species such as IFN-β1b and IFN-β1a wherein one or more methods according to any of the claims 2-31 is used in combination with one or more methods according to any of the claims 32-43.

45. Method according to any of the preceding claims, in which the biological sample is a blood sample and/or is part of a set of blood samples.

46. Method according to any of the preceding claims, for determining the success rate of treatment of relapsing-remitting multiple sclerosis, secondary progressive multiple sclerosis, progressive relapsing multiple sclerosis and/or primary progressive multiple sclerosis.

47. Method according to any of the preceding claims, for determining the success rate of treatment of relapsing-remitting multiple sclerosis.

48. Method according to any of the preceding claims, for determining the success rate of treatment of multiple sclerosis with type 1 interferons.

49. Method according to any of the preceding claims, for determining the success rate of treatment of multiple sclerosis with interferon-beta (IFN-β) species such as IFN-β1b and IFN-β1a.
